# EUROPÄISCHE PATENTSCHRIFT

(11) **EP 2 018 428 B1**
(45) Veröffentlichungstag und Bekanntmachung des Hinweises auf die Patenterteilung: **15.02.2012**
(21) Anmeldenummer: 07724908.4
(22) Anmeldetag: 05.05.2007
(51) Int. Cl.: C12N 15/62

(54) **ANTIENTZÜNDLICHES FUSIONSPROTEIN**
ANTI-INFLAMMATORY FUSION PROTEIN
PROTÉINE DE FUSION ANTI-INFLAMMATOIRE

(30) Priorität: 17.05.2006 DE 102006024113; 13.07.2006 DE 102006033394
(43) Veröffentlichungstag der Anmeldung: 28.01.2009
(73) Patentinhaber: Eberhard-Karls-Universität Tübingen Universitätsklinikum, 72076 Tübingen (DE)
(72) Erfinder: GAWAZ, Meinrad, 72076 Tübingen (DE); DAUB, Karin, 72074 Tübingen (DE)
(74) Vertreter: Findeisen, Marco
(86) Internationale Anmeldenummer: PCT/EP2007/003984
(87) Internationale Veröffentlichungsnummer: WO 2007/131654

(56) Entgegenhaltungen:
- EP-A- 1 046 652
- SIEGEL-AXEL DOROTHEA ET AL: "Specific inhibition of foam cell formation by a soluble CD68-Fc fusion protein" TISSUE ENGINEERING, Bd. 13, Nr. 4, April 2007 (2007-04), Seite 914, XP002456792 & 2ND INTERNATIONAL CONGRESS ON REGENERATIVE BIOLOGY/2ND INTERNATIONAL CONGRESS ON BIO-NANO-INTERFACE; STUTTGART, GERMANY; OCTOBER 09 -11, 2006 ISSN: 1076-3279
- SIEGEL-AXEL ET AL.: "The soluble immunoadhesin CD68-Fc: an innovative anti-atherosclerotic strategy for the specific inhibition of foam cell formation" JOURNAL OF CLINICAL LIPIDOLOGY, Bd. 1, Nr. 5, Oktober 2007 (2007-10), Seite 476, XP002456793

## Beschreibung

Die vorliegende Erfindung betrifft ein Fusionsprotein mit therapeutischem und diagnostischem Potential gegen chronische Gefäßerkrankungen, wie Atherosklerose, ein dieses Fusionsprotein codierendes Nucleinsäuremolekül, eine pharmazeutische und diagnostische Zusammensetzung, die das Fusionsprotein oder Nucleinsäuremolekül aufweist, eine Verwendung des Fusionsproteins oder Nucleinsäuremoleküls zur Herstellung einer pharmazeutischen oder diagnostischen Zusammensetzung, ein Verfahren zur Diagnose von akuten oder chronischen Gefäßerkrankungen, sowie ein Verfahren zur Herstellung des Fusionsproteins.

Die Atherosklerose ist ein hochkomplexer, aktiver pathologischer Prozess, in dessen Zentrum eine inflammatorische Reaktion in den Wänden der blutführenden Gefäße eines betroffenen Individuums steht. Die Entstehung der Atherosklerose, die sogeannte Atherogenese, kann in mehrer Phasen unterteilt werden.

Die frühe Phase der Atherogenese ist gekennzeichnet durch eine sogenannte endotheliale Dysfunktion. Eine Reihe unterschiedlicher Risikofaktoren wie z.B. Rauchen, Übergewicht, körperliche Inaktivität, Hyperlipoproteinämie und Typ-II-Diabetes sowie andere bislang noch nicht identifizierte Faktoren führen zu einer Schädigung des Endothels. Die Permeabilität des Endothels für Lipoproteine und andere zirkulierende Stoffe im Plasma nimmt hierdurch zu. In der Folge werden Endothelzellen aktiviert und exprimieren vermehrt sogenannte Adhäsionsmoleküle auf der Zelloberfläche. Darunter vermitteln vor allem sogenannte Selektine zunächst den temporären Kontakt bestimmter Blutzellen wie Monocyten und T-Lymphocyten mit dem Endothel. Durch eine weitere Gruppe von Adhäsionsmolekülen, den sogenannten Cellular Adhesion Molecules (CAMs), kommt es zur festen Anheftung dieser Zellen an die Gefäßwand. Insbesondere an Gefäßverzweigungen - den Stellen, an denen gehäuft atherosklerotische Läsionen entstehen - spielen zudem mechanische Kräfte eine Rolle. Erhöhte Scherkräfte können die Bildung von endothelialem Stickstoff (NO) verringern. NO wirkt Gefäßerweiternd und besitzt antiinflammatorische Eigenschaften. Darüber hinaus führen erhöhte Scherkräfte zu gesteigerter Bildung von Adhäsionsmolekülen mit den oben geschilderten Folgen.

Im weiteren Verlauf dringen vor allem Monocyten und in geringerem Ausmaß T-Lymphocyten in den subintimalen Raum ein. Dieses Eindringen wird durch eine andere Gruppe von Molekülen, zu denen z.B. das Chemokin Monocyte-Chemoattractant-Protein-1(MCP-1) gehört, vermittelt. Es kommt zur Differenzierung der Monocyten in Makrophagen im subintimalen Raum.

Im geschädigten Endothel kommt es ferner zur Oxidation von Lipoprotein mit geringer Dichte (LDL) und dadurch zur Bildung von oxLDL. Das oxLDL wird in den subintimalen Raum abgegeben, wo es die dort aus Monocyten hervorgegangenen Makrophagen belädt. Diese Makrophagen werden durch die Beladung mit oxLDL zu sogenannten Schaumzellen transformiert, den charakteristischen Zellen der atherosklerotischen Plaques, die als weitere Bestandteile u.a. T-Lymphocyten und aus der Media eingewanderte Glattmuskelzellen enthalten.

Ein solcher atherosklerotische Plaque lagert sich an den Arterienwänden ab und wird dabei von einer stabilisierenden fibrösen Kappe bestehend aus Glattmuskelzellen und extrazellulärer Matrix überzogen. Der atherosklerotische Plaque steht nun im Zentrum einer inflammatorischen Reaktion, bei der es zur Produktion von verschiedensten Entzündungsmediatoren kommt, wie Cytokinen, Chemokinen, Proteasen etc. Dabei kann es zu Gewebenekrosen kommen, in deren Nachbarschaft Kalksalze abgelagert werden. Dadurch kann das Gefäßlumen bis zum völligen Verschluss mit der Folge von Durchblutungsstörungen verengt werden.

Kommt es zur verminderten Bildung von extrazellulärer Matrix durch Glattmuskelzellen und deren Vermehrten Abbau durch degradierende Enzyme kommt es zur Ausdünnung der fibrösen Kappe und der atherosklerotische Plaque wird destabilisiert. Der Plaque kann Aufreißen, wodurch der thrombogene Lipidkern und Kollagen in der Gefäßwand freigelegt werden. Die dadurch bedingte Aktivierung des Hämostasesystems führt zur okkludierenden und nicht-okkludierenden Thrombusbildung, d.h. zur Aktivierung der Gerinnungskaskade, in dessen Zentrum der sogenannte Tissue Factor (TF) steht. Klinisch manifestiert sich die Plaqueruptur mit Thrombusformation als instabile Angina pectoris oder akuter Myokardinfarkt.

Derzeit wird die Atherosklerose in der Regel über die Verabreichung von Lipidsenkern bzw. Statinen behandelt. Hierbei handelt es sich um eine Gruppe von Wirkstoffen, die letztlich die endogene Cholesterinsynthese hemmen. Zu den Statinen zählen u.a. Atorvastatin, Cerivastatin, Fluvastatin, Lovastatin (Mevinolin), Mevastatin (Compactin), Pravastatin und Simvastatin. Diese Substanzen haben auf verschiedene Weise Einfluss auf den Lipidstoffwechsel, z.B. durch kompetitive Hemmung des Schlüsselenzyms der Cholesterinsynthese, der 3-Hydroxy-3-Methylglutaryl-Coenzym-A-Reductase, durch Verminderung der Cholesterinbiosynthese in der Leber, durch Vermehrung der LDL-Rezeptoren auf der Leberzelle und durch Modifikation der Lipoproteinzusammensetzung. Statine haben einen großen Einfluss auf die Zusammensetzung der Serumlipide und führen u.a. zu einer leichten Anhebung der Konzentration von sogenannten "High Density Lipoproteinen" (HDL) und zu einer starken Senkung der LDL-Konzentration. Insgesamt zirkulieren durch die Wirkung der Statine weniger Fette im Blut, so dass die atherosklerotischen Plaques weniger Fette einlagern und das Risiko einer Thrombose und der daraus folgenden Gefährdungen sinkt dadurch.

Obwohl den Statinen noch eine Reihe weiterer positiver Eigenschaften zugeschrieben wird, sind diese auf Grund einer auffälligen Häufung von seltenen, jedoch schwerwiegenden Nebenwirkungen an Muskeln und Nieren im Zusammenhang mit der Einnahme in die Kritik geraten. Deshalb wurde insbesondere der Wirkstoff Cerivastatin (z.B. Lipobay®, Zenas®) in Deutschland im August 2001 vom Markt genommen.

Das Dokument EP 1 046 652 beschreibt ein Fusionsprotein, das eine extrazelluläre Domäne von bovinem LOX-1-Rezeptor und eine Fc-Domäne aufweist und in einem Assay zur Untersuchung der Interaktion von oxidiertem LDL und dem LOX-1 Rezeptor eingesetzt wird.

Vor diesem Hintergrund ist es Aufgabe der vorliegenden Erfindung eine neue Substanz bereitzustellen, die therapeutisches und diagnostisches Potential betreffend die Behandlung und Diagnose von akuten oder chronischen Gefäßerkrankungen, wie der Atherosklerose oder arteriosklerotischen Plaques, aufweist, und mit der die vorstehend genannten Nachteile der bekannten Lipidsenker reduziert oder weitgehend vermieden werden.

Diese Aufgabe wird durch ein Fusionsprotein gelöst, das (a) ein erstes spezifisch an oxidiertes LDL (oxLDL) bindendes Polypeptid sowie (b) ein zweites eine Dimerisierung vermittelndes Polypeptid aufweist, wobei das erste Polypeptid einen Abschnitt oder eine Sequenzvariante von CD68 aufweist, der bzw. die die LDL-Bindefunktion von CD68 aufweist.

Erfindungsgemäß wird unter einem Fusionsprotein ein Hybridprotein bzw. ein artifizielles Protein verstanden, welches *in vitro* aber auch *in vivo* durch im Stand der Technik bekannte molekularbiologische Methoden herstellbar ist. Dazu werden vorzugsweise übliche Expressionsvektoren verwendet, die für das erfindungsgemäße Fusionsprotein codieren. Diese Expressionsvektoren werden in eine geeignete Zelle eingebracht, die dann das Fusionsprotein produziert.

Das erste Polypeptid ist dabei durch geeignete Wahl der Aminosäuresequenz derart gestaltet, dass dieses eine Sekundär- bzw. Tertiärstruktur einnimmt, die selektiv und hochaffin an oxidiertes Low Density Lipoprotein (oxLDL) bindet. Dies ist für ein Proteinsynthesechemiker ein Leichtes, da die räumliche Struktur von modifiziertem LDL im Stand der Technik bekannt ist. Das zweite Polypeptid wird hinsichtlich seiner Aminosäuresequenz derart gestaltet, dass dieses einen Abschnitt eines Proteins aufweist, der in die Vermittlung einer Dimerisierung von zwei separaten Proteinen oder Proteinuntereinheiten involviert ist. Auch diese Maßnahme ist für den Fachmann ein Leichtes, da die Feinstrukturen einschließlich die Aminosäuresequenzen von peptidischen Dimerkomplexen für eine Vielzahl von Proteinen im Stand der Technik ausführlich beschrieben sind. Bekannte dimerbildende in ihrer Sequenz und Struktur bekannte Proteine umfassen G-Proteine, Histone, Interferon y, Interleukin-2-Rezeptor, Hsp90, Tyrosin-Kinasen, IgG-Moleküle etc. Die jeweiligen die Dimerisierung vermittelnden Domänen der genannten Proteine können zur Herstellung des erfindungsgemäßen Fusionsproteins direkt übernommen werden. Allerdings kann es durchaus gewünscht sein, diese Domänen durch gezielte Mutagenese oder auch durch C- und/oder N-terminales Hinzufügen einzelner Aminosäuren zu modifizieren, so dass bspw. die immunologische Wirkung des Fusionsproteins reduziert wird, die besseren Herstellung des Fusionsproteins ermöglicht wird, die Dimerisierungsfunktion jedoch weitgehend erhalten bleibt.

Die der Erfindung zugrunde liegende Aufgabe wird hiermit vollständig gelöst. So haben die Erfinder nämlich erkannt, dass ein erfindungsgemäßes Fusionsprotein nach der Applikation in ein Lebewesen vorzugsweise ausschließlich an vorgeschädigten oder atherosklerotisch veränderten Gefäßarealen angereichert wird, so dass mögliche unspezifische systemische Nebenwirkungen weitgehend vermieden werden können. Das erfindungsgemäße Fusionsprotein fängt dann auf Grund der hohen Affinität für oxidiertes LDL, die durch den ersten Polypeptidabschnitt vermittelt wird, das oxidierte LDL ab. Obgleich auch ein Monomer des Fusionsproteins in der Lage ist oxidierte LDL abzufangen, so wird durch die Dimerisierung von zwei erfindungsgemäßen Fusionsproteinen, die durch den zweiten Polypeptidabschnitt vermittelt wird, die Affinität zu oxidiertem LDL um ein Vielfaches gesteigert.

Durch die Komplexierung des oxidierten LDL wird dieses inaktiviert und die Beladung von Makrophagen und die anschließende Transformation dieser Zellen in Schaumzellen wird damit verhindert bzw. weitgehend reduziert. Dadurch wird bereits in die frühe Phase der Atherogenese eingegriffen. Die Ausbildung von atherosklerotischen Plaques und die o.g. fatalen Auswirkungen auf den Organismus werden deutlich inhibiert und ggf. sogar verhindert. Dabei stellt sich als weiterer besonderer Vorteil dar, dass der Komplex aus Fusionsprotein und oxidiertem LDL auf Grund seines vollständig humanen Ursprungs wenig immunogen ist und bei der "Entsorgung" etwaige Entzündungsreaktionen äußerst schwach ablaufen oder gänzlich ausbleiben.

Das erste Polypeptid ist derart ausgestaltet, dass dieses an oxidiertes LDL (oxLDL) bindet.

Diese Maßnahme hat den Vorteil, dass ein solches Fusionsprotein bereitgestellt wird, das an genau die LDL-Variante bindet und abfängt, die für die Transformation von Makrophagen in Schaumzellen eine entscheidende Rolle spielt. Durch diese erfindungsgemäße Weiterbildung wird demnach mit oxLDL ein Schlüsselfaktor der Atherogenese gezielt in seiner Funktion geschwächt oder sogar ausgeschaltet.

Das erste Polypeptid weist einen Abschnitt oder eine Sequenzvariante auf, der bzw. die die LDL-Bindefunktion von CD68 aufweist.

Nach einer erfindungsgemäßen Weiterbildung weist das erste Polypeptid den Scavengerrezeptor CD68 auf, vorzugsweise die extrazelluläre Domäne von CD68 oder aber ein Fragment oder eine solche Variante der extrazellulären Domäne von CD68 auf, die die LDL-Bindefunktion von CD68 aufweist.

Diese Maßnahme hat den Vorteil, dass als erstes Polypeptid ein solches bereitgestellt wird, welches eine besonders hohe und selektive Affinität zu oxLDL aufweist. CD68, das auch als Makrosialin oder gp110 bezeichnet wird, stellt ein Transmembranglykoprotein dar, das verstärkt in Monocyten und Gewebemakrophagen exprimiert wird. Sowohl die Aminosäuresequenz als auch die codierende Nucleotidsequenz sind im Stand der Technik offenbart; vgl. die NCBI-Datenbankzugriffs-Nrn. AAB25811 oder NP_001242 (Aminosäuresequenz der humanen Variante), Datenbankzugriffs-Nr. NM_001251 oder AAH15557 (Nucleotidsequenz der mRNA der humanen Variante). Die vorstehend identifizierten Aminosäure- und Nucleotidsequenzen sind durch Inbezugnahme Bestandteil der vorliegenden Anmeldung.

Die humane Variante von CD68 betrifft ein 110kD großes Protein, das aus 354 Aminosäuren besteht. CD68 wird zu den sogenannten Scavenger-Rezeptoren gezählt, da es über seine extrazelluläre Domäne an das oxidierte LDL binden kann. CD68 wird natürlicherweise auf der Oberfläche von Monocyten, Makrophagen, neutrophilen und basophilen Zellen sowie großen Lymphocyten exprimiert.

Dabei ist es ferner bevorzugt, wenn das erste Polypeptid nicht die gesamte Aminosäuresequenz von CD68 sondern vielmehr die extrazelluläre Domäne von CD68 aufweist.

Diese Maßnahme hat den besonderen Vorteil, dass für die erfindungsgemäße Funktion des Fusionsproteins unnötige Peptidabschnitte weggelassen werden. Das erfindungsgemäße Fusionsprotetein wird in seiner Größe reduziert, ohne dass dadurch die Positionen 66 bis 960 entspricht. Es versteht sich, dass eine Abgrenzung naturgemäß nicht auf einen Aminosäurerest oder ein Nucleotid genau möglich ist.

Zur Erfüllung der erfindungsgemäßen Funktion muss das Fusionsprotein nicht zwingend die vollständige bzw. identische Aminosäuresequenz von CD68 oder der extrazellulären Domäne von CD68 aufweisen. Vielmehr wird die erfindungsgemäße Funktion des Fusionsproteins auch dann erfüllt, wenn das erste Polypeptid einen Abschnitt oder eine Sequenzvariante von CD68 bzw. der extrazellulären Domäne von CD68 aufweist, der bzw. die jedoch noch die LDL-Bindefunktion von CD68 in ggf. abgeschwächter Form ausübt. Bekanntermaßen werden die proteinogenen Aminosäuren in vier Gruppen unterteilt, nämlich in polare, nicht-polare, saure und basische Aminosäuren. Der Austausch einer polaren Aminosäure gegen eine andere polare Aminosäure, bspw. Glycin gegen Serin, führt in der Regel zu keiner oder nur einer geringfügigen Änderung der biologischen Aktivität des entsprechenden Proteins, so dass ein solcher Aminosäureaustausch das erfindungsgemäße Fusionsprotein in seiner Funktion weitgehend unberührt lässt. Vor diesem Hintergrund erfasst die vorliegende Erfindung auch ein solches Fusionsprotein, das als erstes Polypeptid eine Variante von CD68 oder dessen extrazellulärer Domäne aufweist, bei der eine oder mehrere Aminosäuren einer der genannten Aminosäure-Klassen gegen eine andere Aminosäure der selben Klasse ausgetauscht ist. Eine solche Sequenzvariante ist dabei vorzugsweise zu ca. 70 %, weiter vorzugsweise zu ca. 80 % und höchst vorzugsweise zu ca. 90 bis 95 % homolog zu der Aminosäuresequenz von CD68 bzw. von der extrazellulären Domäne von CD68.

Dabei ist es bevorzugt, wenn das zweite Polypeptid eine Fc-Domäne eines Immunglobulins aufweist, oder aber ein Fragment oder eine Variante der Fc-Domäne aufweist, das bzw. die die Dimerisierungsfunktion der Fc-Domäne aufweist. Mit dieser Maßnahme wird auf vorteilhafte Art und Weise sichergestellt, dass das Fusionsprotein lediglich die Peptidabschnitte aufweist, die für die Dimerisierung von zwei Monomeren des erfindungsgemäßen Fusionsproteins ausreichend sind. "Fc" steht dabei für "fragment crystallizable". Dieses Fragment entsteht durch Papain-Spaltung des IgG-Moleküls neben den beiden Fab-Fragmenten. Die Fc-Domäne besteht aus den gepaarten C_{H}2- und C_{H}3-Domänen einschließlich der Gelenkregion (engl. "hinge region") und enthält den Teil des Immunglobulins, der für die Dimerisierungsfunktion verantwortlich ist. Es versteht sich, dass auch ein Fragment oder eine Variante der Fc-Domäne verwendet werden kann, ohne dass die erfindungsgemäße Funktion des Fusionsproteins beeinträchtigt wird, solange das Fragment bzw. die Variante noch die ggf. abgeschwächte Dimerisierungsfunktion eines Antikörpers aufweist; vgl. vorstehende Ausführungen zum Fragment oder der Variante von CD68, die für das Fragment oder die Variante von Fc gleichermaßen gelten.

Nach einer bevorzugten Weiterbildung weist die Variante der Fc-Domäne eine solche Mutation im Komplement- und Fc-Rezeptorbindebereich auf, die die Immunogenität des erfindungsgemäßen Fusionsproteins reduziert.

Diese Maßnahme hat den Vorteil, dass die Verträglichkeit des erfindungsgemäßen Fusionsproteins weiter erhöht wird. So reicht es nach Erkenntnissen der Erfinder für die erfindungsgemäße Funktion des Fusionsproteins nämlich aus, wenn das zweite Polypeptid lediglich die Dimerisierungsfunktion der Fc-Domäne des Antikörpers aufweist, nicht aber die Effektorfunktionen, die eine Aktivierung des Immunsystems bewirken. Es kann deshalb bspw. auch ein synthetisches Fc-Fragment eingesetzt werden, welches im Komplement- und Fc-Rezeptorbindungsbereich derart mutiert ist, dass eine Aktivierung des Immunsystems weitgehend reduziert wird und ggf. sogar ausbleibt.

Ferner ist es bevorzugt, wenn das Fusionsprotein ein Element aufweist, das das erste Polypeptid mit dem zweiten Polypeptid verbindet.

Diese Maßnahme hat den Vorteil, dass konstruktive Vorraussetzungen geschaffen werden, um die Produktion des erfindungsgemäßen Fusionsproteins mittels verschiedenster Expressionsvektoren zu ermöglichen. Bei dem verbindenden Element kann es sich um eine Aminosäurenabfolge mit beliebiger Zusammensetzung handeln, die vorzugsweise eine bis 100 Aminosäuren aufweist. Bei einem solchen verbindenden Element kann es sich auch um Aminosäuren handeln, die bei der Herstellung des erfindungsgemäßen Fusionsproteins durch Ligation der beiden Polypeptide entstehen.

Nach einer bevorzugten Variante weist das erste Polypeptid des erfindungsgemäßen Fusionsproteins die Aminosäuresequenz SEQ. ID-Nr. 1 aus dem beiliegenden Sequenzprotokoll auf.

Mit dieser Maßnahme wird auf vorteilhafte Art und Weise ein Polypeptid bereitgestellt, das an modifiziertes LDL bzw. oxLDL bindet. Die Aminosäuresequenz SEQ ID-Nr. 1 leitet sich von der Aminosäuresequenz der extrazellulären Domäne des humanen CD68-Proteins ab.

Nach einer bevorzugten Weiterbildung weist das Fusionsprotein ein erstes Polypeptid auf, das von einem Nucleinsäuremolekül codiert wird, das die Nucleotidsequenz SEQ ID-Nr. 2 oder 3 aus dem beiliegenden Protokoll aufweist.

Diese Maßnahme hat den Vorteil, dass bereits eine Codierungssequenz bereitgestellt wird, die für die extrazelluläre Domäne des humanen CD68-Proteins codiert und nach Einbringung in einen Expressionsvektor über Bakterien mittels im Stand der Technik bekannter Verfahren einfach hergestellt werden kann. Die Nucleotidsequenz SEQ ID-Nr. 2 leitet sich von der Nucleotidsequenz ab, die für die extrazelluläre Domäne der humanen Variante des CD68-Proteins codiert. Die Nucleotidsequenz SEQ ID-Nr. 3 berücksichtigt einen in der Bevölkerung vorhandenen Polymorphismus in der extrazellulären Domäne des humanen CD68-Proteins, bei dem ein cag-Basentriplett gegen ein aag-Basentriplett ausgetauscht ist. Eine funktionelle Bedeutung des Polymorphismus ist nicht bekannt.

Es versteht sich, dass nicht nur die Nucleotidsequenz SEQ ID-Nr. 2 oder 3 zur Herstellung der extrazellulären Domäne des CD68-Proteins geeignet ist, sondern auch Varianten hiervon, die auf Grund der Degeneration des genetischen Codes für dasselbe Polypeptid codieren. So ist es bekannt, dass der genetische Code degeneriert ist, da die Anzahl der möglichen Codons größer ist als die Anzahl der Aminosäuren. Für die meisten Aminosäuren gibt es mehr als ein Codon, so dass bspw. Argenin, Leucin und Serin von bis zu sechs Codons codiert wird. In der Regel ist die dritte Codonposition begrenzt oder völlig austauschbar. Vor diesem Hintergrund wird auch ein solches Fusionsprotein bereitgestellt, bei dem das erste Polypeptid von einem Nucleinsäuremolekül codiert wird, das auf Grund der Degeneration des genetischen Codes gegenüber der Nucleotidsequenz SEQ ID-Nr. 2 oder 3 an einzelnen Nucleotidpositionen abweicht, jedoch gleichermaßen für die extrazelluläre Domäne des CD68-Proteins codiert. Vorzugsweise weist eine solche Variante zu der Nucleotidsequenz SEQ ID-Nr. 2 oder 3 ca. 70 % Homologie, weiter vorzugsweise ca. 80 % Homologie und höchst vorzugsweise ca. 90 bis 95 % Homologie auf.

Dabei ist es weiter bevorzugt, wenn das zweite Polypeptid die Aminosäuresequenz SEQ ID-Nr. 4 aus dem beiliegenden Sequenzprotokoll aufweist.

Mit dieser Maßnahme wird auf vorteilhafte Art und Weise bereits ein zweites die Dimerisierung vermittelndes Polypeptid bereitgestellt, das sich von dem Fc-Fragment der humanen IgG1-Variante ableitet und zur Reduzierung der Immunogentät eine Mutation in dem Komplement- und Fc-Rezeptorbindebereich aufweist. Durch gezielte Mutagenese wurde dazu an der Position 331 ein Prolin gegen ein Serin und an den Aminosäurepositionen 234 bis 237 das Tetrapeptid Leu-Leu-Gly-Gly gegen Ala-Ala-Ala-Ala ausgetauscht. Zur Erleichterung der Expression des Peptides wurde dieses Polypeptid ferner gegenüber der natürlichen Sequenz des Fc-Fragmentes von humanem IgG1 für CHO-Zellen codonoptimiert.

Nach einer erfindungsgemäßen Weiterbildung wird das zweite Polypeptid von einem Nucleinsäuremolekül codiert, das die Nucleotidsequenz SEQ ID-Nr. 5 aus dem beiliegenden Sequenzprotokoll aufweist.

Diese Maßnahme hat den Vorteil, dass bereits eine Nucleotidsequenz bereitgestellt wird, die für das vorstehend beschriebene sich von der Fc-Domäne des humanen IgG1-Moleküls ableitende zweite Polypeptid codiert. Nach Einbringung der Codierungssequenz in einen Expressionsvektor werden geeignete Zellen, bspw. CHO-Zellen, mittels im Stand der Technik bekannter Verfahren transformiert, die dann das gewünschte zweite Polypeptid produzieren. Es versteht sich, dass nicht nur die Nucleotidsequenz SEQ ID-Nr. 5 zur Herstellung des zweiten Polypeptids geeignet ist, sondern auch eine Variante hiervon. Es kann vielmehr auch ein solches Nucleinsäuremolekül vorgesehen werden, das auf Grund der Degeneration des genetischen Codes gegenüber der Nucleotidsequenz SEQ ID-Nr. 5 an einzelnen Nucleotidpositionen abweicht, jedoch gleichermaßen für das gewünschte zweite Polypeptid codiert. Vorzugsweise weist eine solche Variante zu der Nucleotidsequenz SEQ ID-Nr. 5 ca. 70 % Homologie, weiter vorzugsweise ca. 80 % Homologie und höchst vorzugsweise 90 bis 95 % Homologie auf.

Besonders bevorzugt ist es, wenn das erfindungsgemäße Fusionsprotein die Aminosäuresequenz SEQ ID-Nr. 6 aus dem beiliegenden Sequenzprotokoll aufweist.

Mit dieser Maßnahme wird bereits eine vollständige Primärstruktur für ein bevorzugtes erfindungsgemäßes Fusionsprotein bereitgestellt, so dass dieses entweder direkt mittels Peptidsynthese oder aber nach Umschreibung in die Codierungssequenz über molekularbiologische Methoden zielgerichtet und einfach hergestellt werden kann. Dabei weist die Aminosäuresequenz SEQ ID-Nr. 6 nicht nur am N-terminalen Ende den Abschnitt für die extrazelluläre Domäne von CD68 und am C-terminalen Ende den Abschnitt für das Fc-Fragment auf, sondern auch ein dazwischenliegendes Element, das aus drei Aminosäuren besteht und die extrazelluläre Domäne von CD68 und das Fc-Fragment miteinander verbindet. Es versteht sich, dass innerhalb der Aminosäuresequenz SEQ ID-Nr. 6 einzelne Aminosäuren einer Klasse gegen Aminosäuren der gleichen Klasse ausgetauscht werden können, wie dies weiter oben dargestellt ist, ohne dass die erfindungsgemäße Funktion des Fusionsproteins deutlich beeinflusst wird. Es versteht sich ferner, dass die Aminosäuresequenz SEQ ID-Nr. 6 an ihrem N- und/oder C-terminalen Ende weitere Aminosäuren aufweisen kann, die lediglich der besseren Expression des Fusionsproteins und Ausschleusung desselben aus den exprimierenden Zellen dienen bzw. lediglich konstruktionsbedingt vorhanden sind. Derartige Aminosäuren können bspw. aus der humanen Ig-kappa-Kette oder aber aus einer multiplen Klonierungsstelle (MCS) stammen.

Es ist ferner bevorzugt, wenn als erfindungsgemäßes Fusionsprotein ein solches bereitgestellt wird, das von einem Nucleinsäuremolekül codiert wird, das die Nucleotidsequenz SEQ ID-Nr. 7 oder 8 aus dem beiliegenden Sequenzprotokoll aufweist.

Diese Maßnahme hat den Vorteil, dass dem Fachmann bereits eine Codierungssequenz angegeben wird, die eine besonders geeignete Ausführungsvariante des erfindungsgemäßen Fusionsproteins codiert. Die Nucleotidsequenz SEQ ID-Nr. 7 weist ebenfalls nicht nur Codierungssequenzen auf, die für die extrazelluläre Domäne von CD68 und für das zweite sich von dem Fc-Fragment ableitende Polypeptid codieren, sondern auch einen dazwischenliegenden Abschnitt, der aus drei Basentripletts besteht, und für das vorstehend genannten verbindende Element codiert. Die Nucleotidsequenz SEQ ID-Nr. 8 berücksichtigt den oben genannten Polymorphismus in der extrazellulären CD68-Domäne, so dass gegenüber der Nucleotidsequenz SEQ ID-Nr. 7 ein cag-Basentriplett gegen ein aag-Basentriplett ausgetauscht ist. Das erfindungsgemäße Nucleinsäuremolekül lässt sich mittels im Stand der Technik bekannter Verfahren in einen Expressionsvektor einbringen, mit dem dann geeignete biologische Zellen, bspw. CHO-Zellen, transformiert werden, welche das gewünschte Fusionsprotein exprimieren. Es versteht sich, dass anstelle der Nucleotidsequenzen SEQ ID-Nr. 7 oder 8 auch Varianten hiervon verwendet werden können, die auf Grund der Degeneration des genetischen Codes für dieselben Fusionsproteine codieren.

Nach einer bevorzugten Weiterbildung weist das erfindungsgemäße Fusionsprotein einen detektierbaren Marker auf.

Diese Maßnahme hat den Vorteil, dass dadurch das Fusionsprotein am Orte seiner Anreicherung *in vitro* aber auch *in vivo* mittels bildgebender Verfahren darstellbar ist und somit zur Identifizierung bspw. von atherosklerotischen Plaques besonders geeignet ist. Erfindungsgemäß wird unter einem detektierbaren Marker eine jegliche Verbindung verstanden, die mittels bildgebender Verfahren identifiziert werden kann. Hierzu zählen Farbindikatoren, wie Farbstoffe mit fluoreszierenden, phosphoreszierenden oder chemilumineszierenden Eigenschaften, AMPPD, CSPD, radioaktive Indikatoren, wie ³²P, ³⁵S, ¹²⁵I, ¹³¹I, ¹⁴C, ³H, nicht radioaktive Indikatoren, wie Biotin oder Digoxigenin, alkalische Phosphatase, Meerrettich-Peroxidase etc. Je nach der Natur des Markers kommen als bildgebende Verfahren die Autoradiographie, Blotting-, Hybridisierungs- oder Mikroskopietechniken in Frage.

Ein weiterer Gegenstand der vorliegenden Erfindung betrifft ein Homodimer, dass das vorstehend identifizierte erfindungsgemäße Fusionsprotein aufweist.

Die Erfinder habe festgestellt, dass die Aktivität des erfindungsgemäßen Fusionsproteins durch die Dimerisierung um ein Vielfaches erhöht wird. Dabei ist von Vorteil, dass bspw. CHO-Zelllinien, die mit einem Expressionsvektor transformiert werden, der für das erfindungsgemäße Fusionsprotein codiert, ein lösliches Dimer sezernieren, bei dem zwei Fusionsproteine über Disulfidbrücken im Bereich des zweiten Polypeptides, bspw. im Bereich der Fc-Domäne, quervernetzt sind. Dabei hat sich herausgestellt, dass bei einem solchen Homodimer im Vergleich zu dem Monomer die Affinität zu modifiziertem LDL, insbesondere zu oxLDL, deutlich erhöht ist. Die Erfinder haben Hinweise, dass sich die Homodimere nach systemischer Applikation in ein Lebewesen rasch und in spezifischer Weise ausschließlich an vorgeschädigten oder atherosklerotisch veränderten Gefäßarealen anreichern und eine schnelle Degradation im Blut sowie mögliche unspezifische systemische Nebenwirkungen weitgehend vermieden werden können. Die Erfinder haben ferner Hinweise darauf, dass das erfindungsgemäße Homodimer zusätzlich eine Deaktivierung oder Blockade des nativen CD68-Rezeptors im Lebewesen bewirken kann, wodurch die LDL-Endocytose durch Makrophagen und Transformation in Schaumzellen zusätzlich verhindert wird. Ein solcher Mechanismus wird im Stand der Technik bereits für andere lösliche Scavenger-Rezeptoren, wie für SR-A1, beschrieben; vgl. Gough et al. (2001), The use of human CD68 transcriptional regulatory sequences to direct high-level expression of class A scavenger receptor in macrophages in vitro and in vivo, Immunology 103, Seiten 351 bis 361. Die Erfinder konnten in Co-Kultivierungsversuchen *in vitro* nachweisen, dass durch Zugabe von steigenden Konzentrationen des erfindungsgemäßen Homodimers eine signifikante Inhibition der Schaumzellbildung erzielt werden kann und verschiedenste Schaumzellfunktionen, wie bspw. die Freisetzung von MMP-9, inhibiert werden können.

Vor diesem Hintergrund betrifft ein weiterer erfindungsgemäßer Gegenstand ein Nucleinsäuremolekül, das für das zuvor identifizierte erfindungsgemäße Fusionsprotein codiert. Das erfindungsgemäße Nucleinsäuremolekül weist vorzugsweise (a) eine erste Nucleotidsequenz auf, die für ein spezifisch an modifiziertes LDL, vorzugsweise an oxLDL, bindendes Polypeptid codiert, und (b) eine zweite Nucleotidsequenz, die für ein eine Dimerisierung vermittelndes Polypeptid codiert. Das erfindungsgemäße Nucleinsäuremolekül weist vorzugsweise die Nucleotidsequenz SEQ ID-Nr. 2 oder 3, Nr. 5 bzw. Nr. 7 oder 8 aus dem beiliegenden Sequenzprotokoll auf, oder eine jeweilige Variante hiervon, die auf Grund der Degeneration des genetischen Codes für dasselbe Polypeptid codiert. Es versteht sich, dass das Nucleinsäuremolekül weitere Nucleotidabschnitte aufweisen kann, die bspw. eine Expression bzw. Herstellung in entsprechend transformierten Zellen ermöglichen bzw. begünstigen. So kann bspw. am 5'-gelegenen Ende ein Leader-Abschnitt aus der Kappa-Kette des IgG-Moleküls sowie daran anschließende konstruktionsbedingte Aminosäuren aus der multiplen Klonierungsstelle (MCS) vorgesehen sein, die jedoch im Translationsprodukt vom gesamten Konstrukt ggf. abgespalten werden und deshalb im erfindungsgemäßen Fusionsprotein nicht zwingend auftauchen.

Ein weiterer Gegenstand der vorliegenden Erfindung betrifft eine pharmazeutische und/oder diagnostische Zusammensetzung, die das erfindungsgemäße Fusionsprotein und/oder das erfindungsgemäße Homodimer und/oder das erfindungsgemäße Nucleinsäuremolekül sowie ggf. einen pharmazeutisch und/oder diagnostisch akzeptablen Träger und ggf. weitere pharmazeutisch und/oder diagnostisch wirksame Zusätze aufweist.

Diagnostisch und pharmazeutisch akzeptable Träger mit ggf. weiteren Zusätzen sind im Stand der Technik allgemein bekannt und werden bspw. in der Abhandlung von Kibbe A., Handbook of Pharmaceutical Excipients, Third Edition, American Pharmaceutical Association and Pharmaceutical Press 2000, beschrieben. Zusätze umfassen erfindungsgemäß jedwede Verbindung oder Zusammensetzung, die für eine diagnostische oder therapeutische Verwendung der Zusammensetzung vorteilhaft sind, worunter Salze, Bindemittel und weitere im Zusammenhang mit der Formulierung von Arzneimitteln verwendete Stoffe fallen.

Ein weiterer Gegenstand der vorliegenden Erfindung betrifft die Verwendung des erfindungsgemäßen Fusionsprotein und/oder des erfindungsgemäßen Homodimers und/oder des erfindungsgemäßen Proteinsäuremoleküls zur Herstellung einer pharmazeutischen und/oder diagnostischen Zusammensetzung zur Behandlung und/oder Diagnose von akuten oder chronischen Gefäßerkrankungen, einschließlich Atherosklerose, atherosklerotische Plaques, Herzinfarkt, Apoplex und periphere, arterielle Verschlusskrankheit (pAVK).

Die Erfindung realisiert sich ferner in einem Verfahren zur Herstellung einer pharmazeutischen und/oder diagnostischen Zusammensetzung zur Behandlung und/oder Diagnose von vorstehend genannten Erkrankungen, das folgende Schritte aufweist: (a) Bereitstellung des erfindungsgemäßen Fusionsproteins und/oder des erfindungsgemäßen Homodimers und/oder des erfindungsgemäßen Nucleinsäuremoleküls; (b) Formulierung in einen pharmazeutisch und/oder diagnostisch akzeptablen Träger, und ggf. (c) Zugabe von weiteren pharmazeutisch und/oder diagnostisch wirksamen Zusätzen.

Ein weiterer Gegenstand der vorliegenden Erfindung betrifft ein Verfahren zur Diagnose von akuten oder chronischen Gefäßerkrankungen, einschließlich Atherosklerose, atherosklerotische Plaques, Herzinfarkt, Apoplex und pAVK, bei einem Lebewesen, das folgende Schritte aufweist: (1) Bereitstellung eines erfindungsgemäßen Fusionsproteins und/oder erfindungsgemäßen Homodimers, das einen detektierbaren Marker aufweist, (2) Einbringen des Fusionsproteins und/oder des Homodimers in das Lebewesen, und (3) Visualisierung der spezifischen Anreicherung des Fusionsproteins in dem Lebewesen mittels bildgebender Verfahren, wie der Positronen-Emissions-Tomographie (PET).

Mittels dieses Verfahrens können auf Grund der selektiven Anreicherung des erfindungsgemäßen Fusionsproteins bzw. erfindungsgemäßen Homodimers atherosklerotische Plaques bzw. hohe Konzentrationen von modifiziertem LDL, einschließlich oxLDL, nachgewiesen werden, was eine zuverlässige Diagnose von entsprechenden Gefäßerkrankungen oder eine Veranlagung hierfür ermöglicht. So sind bislang die Aktivität und die Stabilität von atherosklerotischen Plaques in der im Stand der Technik üblicherweise eingesetzten Angiographie, Kontrastmittelgabe und Vermessung der Gefäßlumenweite nicht gut nachweisbar. Die vorliegende Erfindung schafft hier wirksam Abhilfe.

Ein weiterer Gegenstand der vorliegenden Erfindung betrifft ein Verfahren zur Herstellung des erfindungsgemäßen Fusionsproteins, das folgende Schritte aufweist: (1) Bereitstellen einer ersten Nucleotidsequenz die für ein spezifisch an modifiziertes LDL, vorzugsweise an oxLDL, bindendes Polypeptid codiert, (2) Bereitstellen einer zweiten Nucleotidsequenz, die für ein die Dimerisierung vermittelndes Polypeptid codiert, (3) Ligation der ersten und zweiten Nucleotidsequenz zum Erhalt einer für das Fusionsprotein codierenden Fusionssequenz, (4) Einklonieren der Fusionssequenz in einen Expressionsvektor, (5) Einbringen des Expressionvektors in eine zur Expression geeignete Zelle, (6) Expression des Fusionsproteins in der Zelle, und (7) Isolierung des Fusionsproteins aus der Zelle.

Über dieses Verfahren lässt sich auch das erfindungsgemäße Homodimer herstellen, da geeignete Zellen, wie bspw. HEK- oder CHO-Zellen, die gemäß Schritt (5) transformiert werden, nach der Expression gemäß Schritt (6) bereits ein Homodimer bilden, das im zweiten Polypeptid Disulfidbrücken aufweist, die die beiden erfindungsgemäßen Fusionsproteine miteinander verbinden. Das Homodimer wird von den Zellen in das extrazelluläre Medium sezerniert.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun anhand von Ausführungsbeispielen näher erläutert, die rein exemplarischer Natur sind und die Reichweite der Erfindung nicht einschränken. Dabei wird Bezug genommen auf die beiliegenden Figuren, in denen Folgendes dargestellt ist:
- Fig.1:: Nachweis des fusionierten dimeren CD68-Fc-Fusionsproteins in Westemblot-Analysen.
- Fig. 2:: Differenzierung von CD34⁺-Stammzellen in Schaumzellen nach 10 Tagen der Co-Kultivierung mit Thrombocyten.
- Fig. 3:: (A) Aufnahme von acetyliertem LDL durch Schaumzellen; (B) Inhibition der Schaumzellbildung durch CD68-Fc-Homodimer.
- Fig. 4:: Inhibition der MMP-9-Expression im Überstand von Schaumzellen durch Inkubation mit steigendem CD68-Fc-Konzentrationen.
- Fig. 5:: Spezifischer Fc-ELISA auf atherosklerotischen Plaques *in vitro.*
- Fig. 6:: In-vivo-Applikation von J124-markiertem CD68-Fc in atherosklerotische Apo-E-Mäuse und in Wildtypmäuse.

### Ausführungsbeispiele:

### 1. Nucleotid- und Aminosäuresequenzen

Verwendet werden jeweils die üblichen Einbuchstaben-Codes. Die Aminosäuresequenzen weisen in der Darstellung an ihrem linken Ende das amino- bzw. N-terminale Ende auf, und an ihrem rechten Ende das carboxy- bzw. C-terminale Ende auf. Die Nucleotidsequenzen weisen an Ihrem linken Ende das 5'-Ende auf, und an ihrem rechten Ende das 3'-Ende auf.

### 1.1 Aminosäuresequenz extrazellulären Domäne von CD68 mit Polymorphismus 1 (Glutamin) (SEQ ID-Nr. 1)

### 1.2 Nucleotidsequenz codierend für die extrazelluläre Domäne von CD68, Polymorphismus 1 (SEQ ID-Nr. 2)

Der Polymorphismus 1 ist fett gedruckt.

### 1.3 Nucleotidsequenz codierend für die extrazelluläre Domäne von CD68, Polymorphismus 2 (SEQ ID-Nr. 3)

Der Polymorphismus 2 ist fett gedruckt.

### 1.4 Aminosäuresequenz des zweiten sich von der Fc-Domäne ableitenden und die Dimerisierung vermittelnden Polypeptides (SEQ ID-Nr. 4)

### 1.5 Nucleotidsequenz codierend für das zweite sich von der Fc-Domäne ableitende und die Dimerisierung vermittelnde Polypeptid (SEQ ID-Nr. 5)

### 1.6 Aminosäuresequenz des Gesamt-CD68-Fc-Konstruktes, inklusive verbindendes Element (SEQ ID-Nr. 6)

Das verbindende Element ist unterstrichen. Vor dem verbindenden Element (in Richtung N-Terminus) liegt der Abschnitt der extrazellulären Domäne von CD68, nach dem verbindenden Element (in Richtung C-Terminus) liegt der Abschnitt des sich von der Fc-Domäne ableitenden und die Dimerisierung vermittelnden Polypeptides.

### 1.7 Nucleotidsequenz codierend das Gesamtkonstrukt inklusive das verbindende Element, Polymorphismus 1 (SEQ ID-Nr. 7)

Die Codierungssequenz für das verbindende Element ist unterstrichen. Nach der Codierungssequenz für das verbindende Element (in Richtung N-Terminus) liegt der Abschnitt, der für die extrazelluläre Domäne von CD68 codiert, wobei der Polymorphismus 1 fett gedruckt ist, nach der Codierungssequenz für das verbindende Element (in Richtung C-Terminus) liegt der Abschnitt, der für das zweite die Dimerisierung vermittelnde Polypeptid codiert, das sich von dem Fc-Fragment ableitet.

### 1.8 Nucleotidsequenz codierend das Gesamtkonstrukt inklusive das verbindende Element, Polymorphismus 2 (SEQ ID-Nr. 8)

Die Codierungssequenz für das verbindende Element ist unterstrichen. Nach der Codierungssequenz für das verbindende Element (in Richtung N-Terminus) liegt der Abschnitt, der für die extrazelluläre Domäne von CD68 codiert, wobei der Polymorphismus 2 fett gedruckt ist, nach der Codierungssequenz für das verbindende Element (in Richtung C-Terminus) liegt der Abschnitt, der für das zweite die Dimerisierung vermittelnde Polypeptid codiert, das sich von dem Fc-Fragment ableitet.

### 2. Klonierung des CD68-Fc-Fusionsproteins

Die extrazelluläre Domäne von humanem CD68 wurde mittels Polymerase-Kettenreaktion (PCR) unter Verwendung von spezifischen Primern aus einer frisch präparierten Makrophagen-cDNA-Bibleothek amplifiziert. Dabei wurden an den Enden des Fragmentes neue Restriktionsstellen eingebracht. Das Fragment wurde in ein Plasmid einkloniert, das zur besseren Sekretion eine Leadersequenz der Kappa-Kette von humanem Ig aufweist, mit der die Leadersequenz von CD68 ersetzt wurde. Es wurde ein künstliches Gen synthetisiert, das sich von der Fc-Domäne von humanem IgG1 ableitet. Durch gezielte Mutagenese wurde an der Position 331 ein Prolin gegen ein Serin und an den Aminosäurepositionen 234 bis 237 das Tetrapeptid Leu-Leu-Gly-Gly gegen Ala-Ala-Ala-Ala ausgetauscht. Zur Erleichterung der Expression des Peptides wurde dieses Polypeptid ferner für CHO-Zellen codonoptimiert. Die beiden Fragmente wurden an ihren jeweiligen Enden restriktionsverdaut und legiert, so dass sich der Fc-Teil an den CD68-Teil anschließt. Dabei entstand eine spezifische Verbindungssequenz zwischen den beiden Teilen des Fusionsproteins, die aus drei Aminosäuren besteht. Nachfolgend dargestellt ist die sich hieraus ergebende Fusions-cDNA:

Der IgG-Leader (humane Ig-Kappa-Kette) einschließlich konstruktionsbedingte Aminosäuren aus der MCS sind im Anfangsbereich des Moleküls mittels Großbuchstaben sowie Fettdrucks (IgG-Leader) und unterstrichen (MCS) dargestellt, anschließend folgt die extrazelluläre Domäne von humanem CD68, die mittels Kleinbuchstaben dargestellt ist, ein cag-aag-Polymorphismus ist in Fettdruck dargestellt; hieran anschließend folgt ein Verbindungsabschnitt, der neun Nucleotide aufweist und in Großbuchstaben und unterstrichen dargestellt ist; hieran anschließend folgt die Codierungssequenz für das sich vom Fc-Fragment ableitende Polypeptid, das CHO-Codon-optimiert wurde und im Komplement- und Fc-Rezeptor-Bindungsbereich mutiert ist.

Das codierte Fusionsprotein ist im Folgenden schematisch dargestellt:

Das Leader-Peptid ist mittels Fettdrucks dargestellt (dieser Abschnitt liegt im erfindungsgemäßen Fusionsprotein vorzugsweise nicht mehr oder nur noch Abschnitte hiervon vor), gefolgt von dem MCS-Spacer, der unterstrichen dargestellt ist (dieser Abschnitt kann im erfindungsgemäßen Fusionsprotein ebenfalls fehlen, kann aber auch vorhanden sein), gefolgt von der extrazellulären Domäne von humanem CD68, gefolgt von dem 3-Aminosäure-umfassenden Verbindungselement, das ebenfalls unterstrichen dargestellt ist, gefolgt von dem sich vom Fc-Fragment des humanen IgG1-Moleküls ableitenden Polypeptid, das CHO-Codon-optimiert wurde und im Komplement- und Fc-Rezeptor-Bindungsbereich mutiert ist (hIgG1mut). Der "*" am carboxyterminalen Ende steht für das Stop-Codon.

Die Fusions-cDNA wurde erneut restriktionsverdaut und in einen pcDNA5-Plasmidvektor (Invitrogen) mittels klassischer Klonierung eingebracht. Der resultierende Plasmidvektor wird als pcDNA5-FRT-CD68-Fc-opt bezeichnet.

### 3. Herstellung von CHO-Zellen, die stabil das CD68-Fc-Fusionsprotein exprimieren

Flp-In™-CHO-Zellen (Invitrogen) wurden bei einer Konfluenz von 70 % im Verhältnis von 9:1 mit den Plasmiden pOG44:pcDNA5-FRT-CD68-Fc-opt (beide Invitrogen) cotransfiziert. 24 Stunden nach der Transfektion wurden die Zellen gewaschen und frisches Medium wurde hinzugegeben. 48 Stunden nach der Transfektion wurden die Zellen 1:20 in frisches Medium enthaltend 500 mg/ml Hygromycin überführt. Hygromycin-resistente Foci wurden isoliert und expandiert.

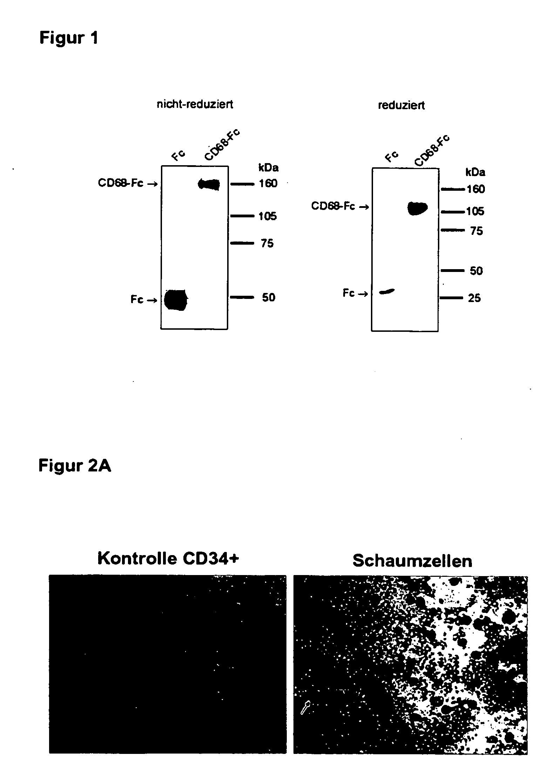

Die expandierten Transformanten wurden auf die Expression des CD68-Fc-Fusionsproteins (Fig. 1: CD68-Fc) mittels Westernblot-Analysen (SDS-PAGE) unter Verwendung von Antikörpern analysiert, die gegen humanes Fc gerichtet waren. Verwendet wurde ein sekundärer Anti-human-Fc-Antikörper. Als Kontrolle diente Fc-Protein (Fig. 1: Fc), das keine extrazelluläre CD68-Domäne aufwies. Dabei zeigte sich für CD68-Fc unter nicht-reduzierenden Bedingungen eine spezifische Bande bei 160 kDa (Fig.1, links), unter reduzierenden Bedingungen bei 115 kDa (Fig. 1, rechts).

Ferner wurde ein quantitativer Nachweis mit Hilfe eines humanen IgG-ELISA durchgeführt. Dabei ließ sich im Zellkulturüberstand der produzierenden CHO-Zelllinie eine CD68-Fc-Konzentration von etwa 2 mg/ml nachweisen, während sich bei Wildtyp-Zellen kein CD68-Fc-Fusionsprotein finden ließ.

### 4. Reinigung des CD68-Fc-Fusionsproteins

Die CHO-Zellen, die das CD68-Fc-Fusionsprotein stabil exprimieren, wurden kultiviert. Drei Tage nach der Infektion wurde der Kulturüberstand bei 3800 g für 30 min. bei 4°C abzentrifugiert und durch einen Filter mit einer Porengröße von 0,2 µm filtriert. Das CD68-Fc-Fusionsprotein wurde durch Zugabe von 1,2 Volumina Ammoniumsulfat (761 g/1) und Schütteln über Nacht bei 4°C präzipitiert.

Die Proteine wurden durch Zentrifugation bei 3000 g für 30 min. bei 4°C pelletiert, in 0,1 Volumina PBS gelöst und über Nacht bei 4°C in PBS dialysiert. Die Proteinlösung wurde durch Zentrifugation bei 14000 g für 30 min. bei 4°C und Filtration durch einen Filter mit einer Porengröße von 0,2 µm geklärt und auf eine Protein-A-Säule (HiTrap™) Protein A HP, (Amersham Pharmacia Wiotech AB, Upsalla, Schweden) geladen, die zuvor mit Bindepuffer (20 mM Natriumphosphat-Puffer pH 7,0, 0,02 % NaN₃) äquilibriert wurde. Die Säule wurde mit Bindepuffer bis zu einer OD₂₈₀ < 0,01 gewaschen und mit Elutionspuffer (100 mM Glycin pH 2,7) eluiert.

Die eluierten Fraktionen von jeweils 900 µl wurden mit 100 µl Neutralisierungspuffer (1 M Tris-HCl pH 9,0, 0,02 % NaN₃) neutralisiert, gepoolt, über Nacht in PBS bei 4°C dialysiert, aliquotiert und bei -20°C eingefroren. Die Säule wurde mit Bindepuffer neutralisiert, mit 20 % (v/v) Ethanol gewaschen und in einem Kühlschrank gelagert.

### 5. Differenzierung von CD34⁺-Stammzellen in Schaumzellen durch Co-Kultivierung mit Thrombocyten

Zur Isolierung von humanen Thrombocyten wurde gesunden Probanten venöses Blut abgenommen und in Acid-Citrat-Dextrose(ACD)-Puffer gesammelt. Nach der Zentrifugation bei 430 g für 20 min. wurde das mit Thrombocyten angereicherte Plasma ["Platelet-Rich Plasma (PRP)"] abgenommen, zu Tyrodes-HEPES-Puffer (2,5 mM HEPES, 150 mM NaCl, 1 mM HCl, 2,5 mM NaHCO₃, 0,36 mM NaH₂PO₄, 5,5 mM Glucose, 1 mg/ml BSA, pH 6,5) gegeben und bei 900 g für 10 min. zentrifugiert. Nach der Entfernung des Überstandes wurde das erhaltene Thrombocyten-Pellet in Tyrodes-HEPES-Puffer (pH 7,4) resuspendiert.

Dieses Verfahren führt zu einer hohen Reinheit der Thrombocyten ohne messbare Kontaminationen durch polymorphkernige Zellen oder Monocyten, was durch die Abwesenheit von CD14 (Durchflusscytometrie und Myeloperoxydase-ELISA) verifiziert wurde. Das vorstehende Verfahren ist beschrieben in Langer H. et al. (2006; elektronisch 2005), Adherent platelets recruit and induce differentiation of murine embryonic endothelial progenitor cells to mature endothelial cells in vitro, Circ. Res. 98(2): e2-10.

Humane CD34⁺-Zellen wurden aus humanem Nabelschnurblut isoliert. Dieses wurde nach Genehmigung des lokalen Ethikkomitees von gesunden Frauen unmittelbar nach der Geburt eines Kindes erhalten. Die isolierten Zellen waren zumindest zu 95 % positiv für CD34⁺, was mittels Durchflusscytometrie-Analyse nach der jeweiligen Isolierung bestätigt wurde. Humane mononukleare Zellen wurden durch Dichtegradientenzentrifugation auf einer Biocoll-Separierungslösung (BIOCROM Berlin, Deutschland) bei 600 g für 15 min. ebenfalls aus Nabelschnurblut erhalten. CD34⁺-Zellen wurden durch Immunaffinitätsselektion angereichert (CD34 Progenitor Cell Isolation Kit; Milteenyi Biotec, Bergisch Glattbach, Deutschland), und zwar gemäß der Anleitung des Herstellers. Die Zellen wurden nachfolgend auf 96-Well-Platten inkubiert, die mit 0,2 % Gelatine beschichtet waren. Zur Zellkultivierung wurden IMDM mit Glutamax, versetzt mit 5% hitzeinaktiviertem fötalem Kälberserum, 100 mg/ml Penicillin-Streptomycin, 1 % MEM-Vitaminen und 1 % nicht essentiellen Aminosäuren verwendet, die sämtlich von Gibco (Invitrogen, Karlsruhe, Deutschland) bezogen wurden. CD34⁺-Vorläuferzellen (50.000 Zellen) wurden mit Thrombocyten (2 x 10⁸-ml) in 96-Well-Platten, die mit 0,2 % Gelatine vorbeschichtet waren, bei 37°C und 5 % CO₂ für 10 Tage co-kultiviert. Die Entwicklung von Schaumzellen wurde in sechs Fenstern mittels Phasenkontrastmikroskopie gezählt.

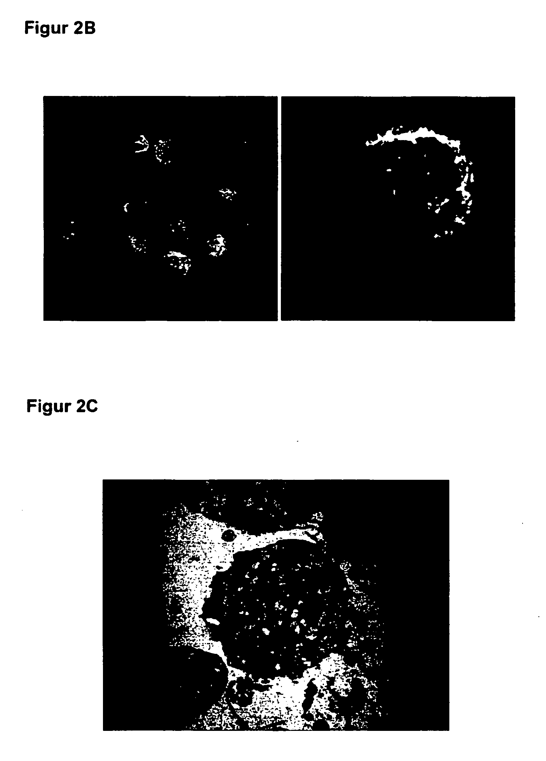

Dabei zeigte sich in der Phasenkontrastmikroskopie eine Differenzierung von CD34⁺-Stammzellen in Schaumzellen, wie dies in Fig. 2A dargestellt ist. In der linken Aufnahme sind Kontroll-CD34⁺-Zellen ohne Schaumzellbildung, in der rechten Aufnahme ist die Schaumzellbildung in Anwesenheit von Thrombocyten (Pfeil) gezeigt. Dass die durch Cokultivierung von CD34⁺-Zellen und Thrombocyten entstandenen "Riesenzellen" tatsächlich Schaumzellen darstellen, wird durch positive Immunfluoreszenzfärbung des Scavenger-Recepturs CD68 (Fig. 2B) und transmissionselektronenmikroskopische Aufnahmen, die eine Schaumzelle darstellen (Fig. 2C), belegt.

### 6. Funktionelle Charakterisierung der gewonnen Schaumzellen

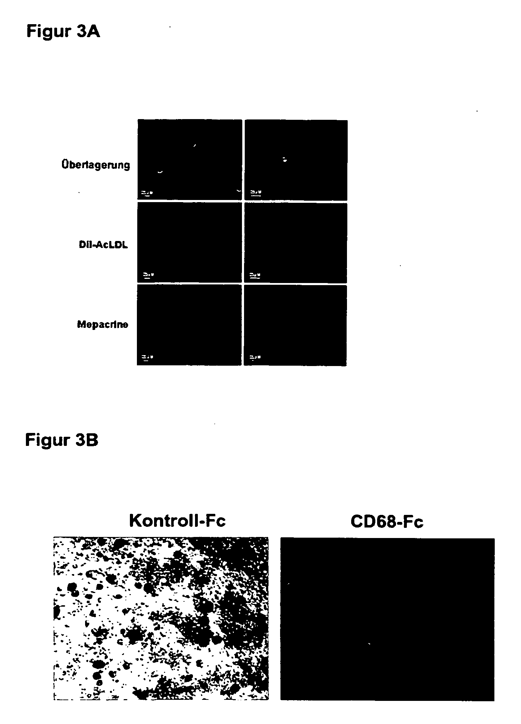

Um die *in vitro* gebildeten Schaumzellen funktionell charakterisieren zu können, wurde überprüft, ob diese acetyliertes LDL (acLDL) aufnehmen können. Dazu erfolgte eine Inkubation der gewonnenen Schaumzellen mit fluoreszenzmarkiertes acLDL (Dil-AcLDL) und eine Anfärbung der Dichtegranula mit Mepacrine. Eine anschließende confokale lasermikroskopische Aufnahme demonstriert eindeutig die Aufnahme von acLDL in die gewonnenen Schaumzellen und damit deren Funktionalität; vgl. Fig. 3A, Maßstabsbalken 25 µm.

Ferner wurde die Methode der ROS ("Reactive Oxigen Species")-Messung verwendet. Charakteristischerweise dringen während eines Entzündungsprozesses Monocyten in die Gefäßwand ein, differenzieren sich dort zu Makrophagen und produzieren Cytokine, Proteasen, wie die Matrixmetalloproteinase (MMP) und Komplementfaktoren, aber auch freie oxidierte Radikale, d.h. ROS. Für die durch Co-Inkubation von CD34⁺-Stammzellen mit Thrombocyten *in vitro* entstandenen Schaumzellen wurde festgestellt, dass diese ROS freisetzen und auch Matrixmetalloproteinase 9 (MMP-9) produzieren. Auch dadurch wurde demonstriert, dass funktionelle Schaumzellen gewonnen wurden.

### 7. Inhibition der Schaumzellbildung und -funktionen durch das CD68-Fc-Fusionsprotein

Schaumzellen wurden durch 10tägige Co-Inkubation von CD34⁺-Stammzellen mit Thrombocyten *in vitro* erzeugt. Ein Teil der Kulturen wurde mit CD68-Fc-Homodimeren behandelt, ein anderer Teil zur Kontrolle mit reinem Fc. Anschließend wurde mikroskopisch untersucht, ob durch die Inkubation mit CD68-Fc-Homodimeren die Schaumzellbildung gehemmt wurde. Das Ergebnis ist in Fig. 3B dargestellt. Dabei zeigte sich, dass eine Behandlung mit Fc (linke Teilabbildung) die Schaumzellbildung unbeeinflusst lässt, wohingegen die Behandlung mit CD68-Fc-Homodimeren (rechte Teilabbildung) die Schaumzellbildung inhibierte.

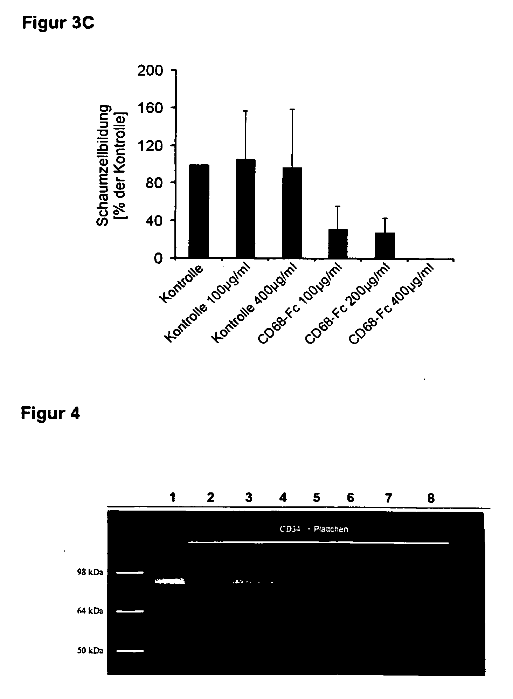

In Fig. 3C ist die Abhängigkeit der Reduktion der Schaumzellenbildung von Dosis an CD68-Fc-Homodimer dargestellt. Bei einer Konzentration von 400 µg/ml CD68-Fc-Homodimer ist die Schaumzellbildung praktisch vollständig inhibiert. Als Kontrolle wurde wiederum reines Fc eingesetzt.

Ferner wurde in einem weiteren Experiment festgestellt, dass die MMP-9-Expression im Überstand von Schaumzellen durch Inkubation mit steigendem CD68-Fc-Homodimer-Konzentrationen im Vergleich zu Kontrollprotein inhibiert wird. Das Ergebnis dieses Experimentes ist in Fig. 4 dargestellt. Die Banden entsprechen dem Expressionsniveau für MMP-9. Aufgetragen sind in den jeweiligen Spuren folgende Ansätze: 1. Kontroll-CD34, 2. Kontroll-CD34, 3. Kontrollprotein 100g/ml, 4. Kontrollprotein 400µg/ml, 5. CD68-Fc-Homodimer 100g/ml, 6. CD68-Fc-Homodimer 200g/ml, 7. CD68-Fc-Homodimer 400g/ml, 8. Fluvastatin 1µM. Dabei zeigte sich, dass die Anwesenheit von CD68-Fc-Homodimer (Spuren 5, 6 und 7) zu einer deutlichen Hemmung der MMP-9-Bildung durch die Schaumzellen führt, ähnlich wie durch das gut charakterisierte Statin Fluvastatin (Spur 8).

### 8. CD68-Fc-Fusionsprotein als diagnostisches Agens

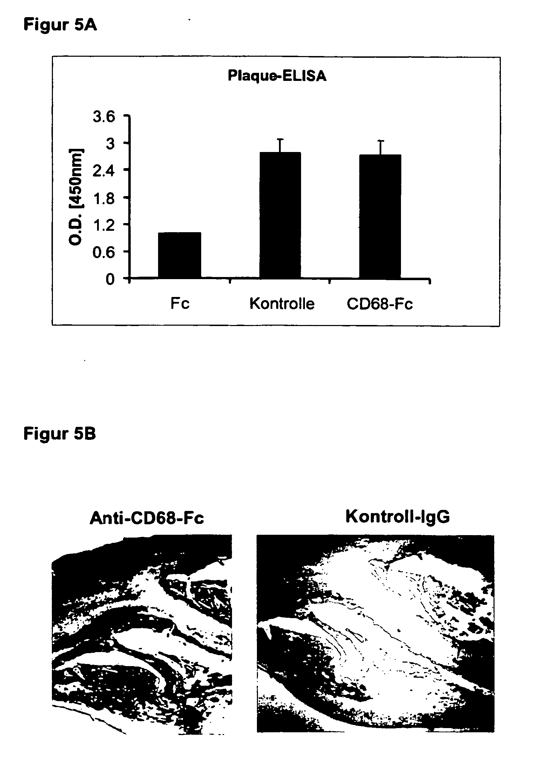

Atherosklerotische Plaques aus der Carotis von Patienten, die im Rahmen einer Operation entnommen wurden, wurden zerkleinert, suspendiert, in Kulturplatten überführt und angetrocknet. Danach wurde ein spezifischer ELISA gegen den Fc-Teil des CD68 bzw. GPVI bzw. reines Fc durchgeführt. Das Ergebnis ist in Fig. 5A dargestellt. Parallel hierzu wurden immunhistologische Untersuchungen von humanem Gewebe aus Thrombenarteriektomiepräparaten der Arteria carotis durchgeführt. Das Ergebnis ist in Fig. 5B dargestellt.

In beiden Fällen zeigt sich, dass eine signifikante Bindung von CD68-Fc-Fusionsprotein an atherosklerotisches Plaquegewebe stattfindet im Vergleich zu Kontroll-Fc Protein (Fc) welches keine spezifische Bindung an Plaquegewebe aufweist. Als positive Kontrolle wurde ein Protein verwendet welches an Kollagenstrukturen in humanem Plaquegewebe bindet (Kontrolle).

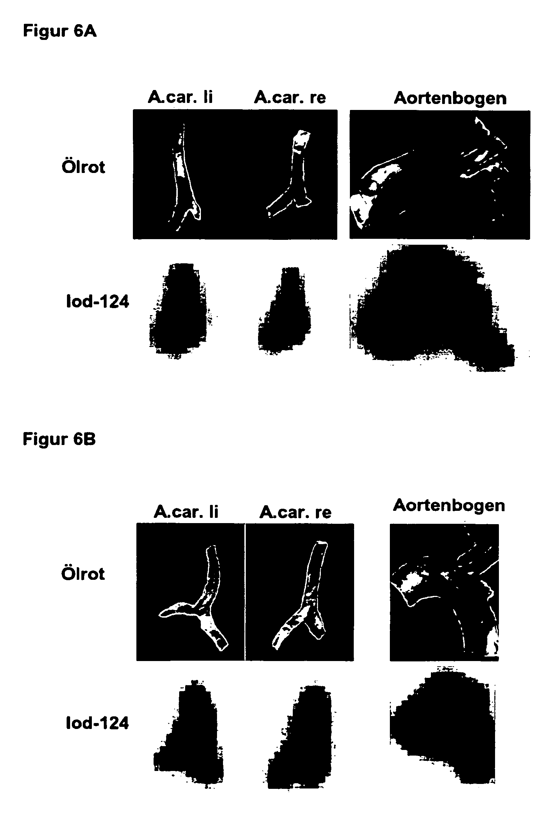

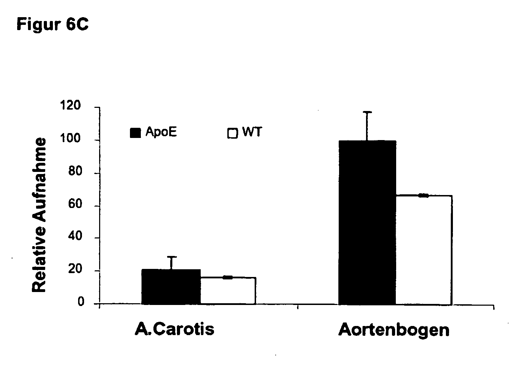

Ferner wurden eine in-vivo-Applikation von J124-markiertem CD68-Fc in atherosklerotische Apo-E-Mäuse und in Wildtypmäuse durchgeführt. J124-markiertes CD68-Fc wurde in 24-Wochen alte atherosklerotische Mäuse appliziert oder in Kontroll-Wildtypmäuse ohne wesentliche Atherosklerose. Danach wurden die Organe entnommen und die A. carotis bds. und der Aortenbogen autoradiographisch *ex vivo* untersucht. Das Ergebnis ist in Fig. 6 gezeigt.

Wie in Teillabbildung (A) dargestellt, zeigt sich eine deutlich vermehrte Radioaktivität in atherosklerotischen Gefäßsegmenten im Vergleich zu nicht-atherosklerotischen Gefäßen. Eine Ölrotfärbung und korrespondierende Autoradiographie ist in Teilabbildung (B) gezeigt. Teilabbildung (C) zeigt eine quantitative Auswertung der Autoradiographie.

Es zeigt sich dass atherosklerotische Gefäße (Aortenbogen) entnommen aus den ApoE-Mäusen eine erhebliche Mehranreicherung von J124-markiertem CD68-Fc-Fusionsprotein aufweisen im Vergleich zu nicht-atherosklerotischen Gefäßen entnommen aus Wildtyp-Mäusen. Keine signifikante Mehranreicherung bei ApoE-Mäusen von CD68-Fc-Fusionsprotein findet sich im Bereich der A. carotis im Vergleich zum Wildtyp.

## Patentansprüche

1. Fusionsprotein, aufweisend:
(a) ein erstes spezifisch an oxidiertes LDL (oxLDL) bindendes Polypeptid, und
(b) ein zweites eine Dimerisierung vermittelndes Polypeptid,
**dadurch gekennzeichnet, dass** das erste Polypeptid einen Abschnitt oder eine Sequenzvariante von CD68 aufweist, der bzw. die die LDL-Bindefunktion von CD68 aufweist.

2. Fusionsprotein nach Anspruch 1, **dadurch gekennzeichnet, dass** das erste Polypeptid CD68 aufweist, vorzugsweise die extrazelluläre Domäne von CD68 oder ein Fragment oder eine Variante davon aufweist, das bzw. die die LDL-Bindefunktion von CD68 aufweist.

3. Fusionsprotein nach Anspruch 1 oder 2, **dadurch gekennzeichnet, dass** das zweite Polypeptid eine Fc-Domäne eines Immunglobulins oder ein Fragment oder eine Variante davon aufweist, das bzw. die die Dimerisierungsfunktion der Fc-Domäne aufweist.

4. Fusionsprotein nach Anspruch 3, **dadurch gekennzeichnet, dass** die Variante der Fc-Domäne eine solche Mutation im Komplement- und Fc-Rezeptorbindebereich aufweist, die die Immunogenität des Fusionsproteins reduziert.

5. Fusionsprotein nach einem der Ansprüche 1 bis 4, **dadurch gekennzeichnet, dass** das Fusionsprotein ferner ein Element aufweist, der das erste Polypeptid mit dem zweiten Polypeptid verbindet.

6. Fusionsprotein nach einem der Ansprüche 1 bis 5, **dadurch gekennzeichnet, dass** das erste Polypeptid die Aminosäuresequenz SEQ ID-Nr. 1 aus dem beiliegenden Sequenzprotokoll aufweist.

7. Fusionsprotein nach einem der Ansprüche 1 bis 6, **dadurch gekennzeichnet, dass** das erste Polypeptid von einem Nucleinsäuremolekül codiert wird, das die Nucleotidsequenz SEQ ID-Nr. 2 oder 3 aus dem beiliegenden Sequenzprotokoll, oder Varianten hiervon aufweist, die aufgrund der Degeneration des genetischen Codes für dasselbe Polypeptid codieren.

8. Fusionsprotein nach einem der Ansprüche 1 bis 7, **dadurch gekennzeichnet, dass** das zweite Polypeptid die Aminosäuresequenz SEQ ID-Nr. 4 aus dem beiliegenden Sequenzprotokoll aufweist.

9. Fusionsprotein nach einem der Ansprüche 1 bis 8, **dadurch gekennzeichnet, dass** das zweite Polypeptid von einem Nucleinsäuremolekül codiert wird, das die Nucleotidsequenz SEQ ID-Nr. 5 aus dem beiliegenden Sequenzprotokoll, oder eine Variante hiervon aufweist, die aufgrund der Degeneration des genetischen Codes für dasselbe Polypeptid codiert.

10. Fusionsprotein nach einem der Ansprüche 1 bis 9, **dadurch gekennzeichnet, dass** es die Aminosäuresequenz SEQ ID-Nr. 6 aus dem beiliegenden Sequenzprotokoll aufweist.

11. Fusionsprotein nach einem der Ansprüche 1 bis 10, **dadurch gekennzeichnet, dass** es von einem Nucleinsäuremolekül codiert wird, das die Nucleotidsequenz SEQ ID-Nr. 7 oder 8 aus dem beiliegenden Sequenzprotokoll, oder Varianten hiervon aufweist, die aufgrund der Degeneration des genetischen Codes für dasselbe Fusionsprotein codieren.

12. Fusionsprotein nach einem der Ansprüche 1 bis 11, **dadurch gekennzeichnet, dass** das Fusionsprotein ferner einen detektierbaren Marker aufweist.

13. Homodimer, das das Fusionsprotein nach einem der Ansprüche 1 bis 12 aufweist.

14. Nucleinsäuremolekül, das für das Fusionsprotein nach einem der Ansprüche 1 bis 12 codiert.

15. Nucleinsäuremolekül nach Anspruch 14, aufweisend:
(a) eine erste Nucleotidsequenz, die für ein spezifisch an oxLDL bindendes Polypetid codiert, und
(b) eine zweite Nucleotidsequenz, die für ein eine Dimerisierung vermittelndes Polypeptid codiert,
**dadurch gekennzeichnet, dass** das erste Polypeptid die LDL-Bindefunktion von CD68 aufweist.

16. Nucleinsäuremolekül nach Anspruch 14 oder 15, **dadurch gekennzeichnet, dass** es die Nucleotidsequenz SEQ ID-Nr. 2 oder 3, aus dem beiliegenden Sequenzprotokoll, oder Varianten hiervon aufweist, die aufgrund der Degeneration des genetischen Codes für dasselbe Polypeptid codieren.

17. Nucleinsäuremolekül nach einem der Ansprüche 14 bis 16, **dadurch gekennzeichnet, dass** es die Nucleotidsequenz SEQ ID-Nr. 5 aus dem beiliegenden Sequenzprotokoll, oder eine Variante hiervon aufweist, die aufgrund der Degeneration des genetischen Codes für dasselbe Polypeptid codiert.

18. Nucleinsäuremolekül, nach einem der Ansprüch 14 bis 17, **dadurch gekennzeichnet, dass** es die Nucleotidsequenz SEQ ID-Nr. 7 oder 8 aus dem beiliegenden Sequenzprotokoll, oder Varianten hiervon aufweist, die aufgrund der Degeneration des genetischen Codes für dasselbe Polypeptid codieren.

19. Pharmazeutische und/oder diagnostische Zusammensetzung, die das Fusionsprotein nach einem der Ansprüche 1 bis 12 und/oder das Homodimer nach Anspruch 13 und/oder das Nucleinsäuremolekül nach einem der Ansprüche 14 bis 18 und ggf. einen pharmazeutisch und/oder diagnostisch akzeptablen Träger sowie ggf. weitere pharmazeutisch und/oder diagnostisch wirksame Zusätze aufweist.

20. Verwendung des Fusionsproteins nach einem der Ansprüche 1 bis 12 und/oder des Homodimers nach Anspruch 13 und/oder des Nucleinsäuremoleküls nach einem der Ansprüche 14 bis 18 zur Herstellung einer pharmazeutischen und/oder diagnostischen Zusammensetzung zur Behandlung und/oder Diagnose von akuten oder chronischen Gefäßerkrankungen, einschließlich Atherosklerose und arteriosklerotische Plaques.

21. Verfahren zur Herstellung des Fusionsproteins nach einem der Ansprüche 1 bis 12, das folgende Schritte aufweist:
(1) Bereitstellen einer ersten Nucleotidsequenz, die für ein spezifisch an oxLDL bindendes Polypetid codiert, das die LDL-Bindefunktion von CD68 aufweist,
(2) Bereitstellen einer zweiten Nucleotidsequenz, die für ein eine Dimerisierung vermittelndes Polypeptid codiert,
(3) Ligation der ersten und zweiten Nucleotidsequenz zum Erhalt einer für das Fusionsprotein codierenden Fusionssequenz,
(4) Einklonieren der Fusionssequenz in einen Expressionsvektor,
(5) Einbringen des Expressionvektors in eine zur Expression geeignete Zelle,
(6) Expression des Fusionsproteins in der Zelle, und
(7) Isolierung des Fusionsproteins aus der Zelle.

## Claims

1. Fusion protein, comprising:
(a) a first polypeptide which specifically binds to oxidized LDL (oxLDL), and
(b) a second polypeptide which mediates a dimerization,
**characterized in that** the first polypeptide comprises a segment or a sequence variant of CD68, which comprises the LDL binding function of CD68.

2. Fusion protein of claim 1, **characterized in that** the first polypeptide comprises CD68, preferably the extracellular domain of CD68 or a fragment or a variant thereof, which comprises the LDL binding function of CD68.

3. Fusion protein of claim 1 or 2, **characterized in that** the second polypeptide comprises a Fc domain of an immunoglobulin or a fragment or a variant thereof, which comprises the dimerization function of the Fc domain.

4. Fusion protein of claim 3, **characterized in that** the variant of the Fc domain comprises a mutation in the complement and Fc receptor binding area, which reduces the immunogenicity of the fusion protein.

5. Fusion protein of any of claims 1 to 4, **characterized in that** the fusion protein further comprises an element which connects the first polypeptide to the second polypeptide.

6. Fusion protein of any of claims 1 to 5, **characterized in that** the first polypeptide comprises the amino acid sequence SEQ ID No. 1 from the enclosed sequence listing.

7. Fusion protein of any of claims 1 to 6, **characterized in that** the first polypeptide is encoded by a nucleic acid molecule which comprises the nucleotide sequence SEQ ID No. 2 or 3 from the enclosed sequence listing or variants thereof, which, due to the degeneration of the genetic code, encode the same polypeptide.

8. Fusion protein of any of claims 1 to 7, **characterized in that** the second polypeptide comprises the amino acid sequence SEQ ID No. 4 from the enclosed sequence listing.

9. Fusion protein of any of claims 1 to 8, **characterized in that** the second polypeptide is encoded by a nucleic acid molecule which comprises the nucleotide sequence SEQ ID No. 5 from the enclosed sequence listing or a variant thereof, which, due to the degeneration of the genetic code, encodes the same polypeptide.

10. Fusion protein of any of claims 1 to 9, **characterized in that** it comprises the amino acid sequence SEQ ID No. 6 from the enclosed sequence listing.

11. Fusion protein of any of claims 1 to 01, **characterized in that** it is encoded by a nucleic acid molecule which comprises the nucleotide sequence SEQ ID No. 7 or 8 from the enclosed sequence listing or variants thereof, which, due to the degeneration of the genetic code, encode the same fusion protein.

12. Fusion protein of any of claims 1 to 11, **characterized in that** the fusion protein further comprises a detectable marker.

13. Homodimer which comprises the fusion protein of any of claims 1 to 12.

14. Nucleic acid molecule which encodes the fusion protein of any of claims 1 to 12.

15. Nucleic acid molecule of claim 14, comprising:
(a) a first nucleotide sequence which encodes a polypeptide which specifically binds to oxLDL, and
(b) a second nucleotide sequence which encodes a polypeptide which mediates a dimerization,
**characterized in that** the first polypeptide comprises the LDL binding function of CD68.

16. Nucleic acid molecule of claim 14 or 15, **characterized in that** it comprises the nucleotide sequence SEQ ID No. 2 or 3 from the enclosed sequence listing or variants thereof, which, due to the degeneration of the genetic code, encode the same polypeptide.

17. Nucleic acid molecule of any of claims 14 to 16, **characterized in that** it comprises the nucleotide sequence SEQ ID-No. 5 from the enclosed sequence listing or a variant thereof, which, due to the degeneration of the genetic code, encodes the same polypeptide.

18. Nucleic acid molecule of any of claims 14 to 17, **characterized in that** it comprises the nucleotide sequence SEQ ID-No. 7 or 8 from the enclosed sequence listing or variants thereof, which, due to the degeneration of the genetic code, encode the same polypeptide.

19. Pharmaceutical and/or diagnostic composition which comprises the fusion protein of any of claims 1 to 12 and/or the homodimer of claim 14 and/or the nucleic acid molecule of any of claims 14 to 18 and, if applicable, a pharmaceutically and/or diagnostically acceptable carrier as well as, if applicable, further pharmaceutically and/or diagnostically active additives.

20. Use of the fusion protein of any of claims 1 to 12 and/or the homodimer of claim 13 and/or the nucleic acid molecule of any of claims 14 to 18 for the preparation of a pharmaceutical and/or diagnostic composition for the treatment and/or the diagnosis of acute or chronic vascular diseases, including atherosclerosis and atherosclerotic plaques.

21. Method for the preparation of the fusion protein of any of claims 1 to 12, comprising the following steps:
(1) providing of a first nucleotide sequence which encodes a polypeptide which specifically binds to oxLDL,
(2) providing of a second nucleotide sequence which encodes a polypeptide which mediates a dimerization,
(3) ligation of the first and the second nucleotide sequence to obtain a fusion sequence encoding the fusion protein,
(4) cloning the fusion sequence into an expression vector,
(5) introducing the expression vector into a cell suitable for the expression,
(6) expression of the fusion protein in the cell, and
(7) isolation of the fusion protein from the cell.

## Revendications

1. Protéine de fusion, présentant :
(a) un premier polypeptide se liant spécifiquement au LDL oxydé (oxLDL), et
(b) un deuxième polypeptide entraînant une dimérisation,
**caractérisée en ce que** le premier polypeptide présente un segment ou une variante de séquence de CD68 qui présente la fonction de liaison du LDL de CD68.

2. Protéine de fusion selon la revendication 1, **caractérisée en ce que** le premier polypeptide présente CD68, de préférence, présente le domaine extracellulaire de CD68 ou un fragment ou une variante de celui-ci qui présente la fonction de liaison du LDL de CD68.

3. Protéine de fusion selon la revendication 1 ou 2, **caractérisée en ce que** le deuxième polypeptide présente un domaine Fc d'une immunoglobuline ou un fragment ou une variante de celui-ci, qui présente la fonction de dimérisation du domaine Fc.

4. Protéine de fusion selon la revendication 3, **caractérisée en ce que** la variante du domaine Fc présente une mutation dans le domaine du complément et de liaison du récepteur de Fc qui réduit l'immunogénicité de la protéine de fusion.

5. Protéine de fusion selon l'une des revendications 1 à 4, **caractérisée en ce que** la protéine de fusion présente en outre un élément qui lie le premier polypeptide au deuxième polypeptide.

6. Protéine de fusion selon l'une des revendications 1 à 5, **caractérisée en ce que** le premier polypeptide présente la séquence d'acides aminés SEQ ID N° : 1 du protocole de séquence joint.

7. Protéine de fusion selon l'une des revendications 1 à 6, **caractérisée en ce que** le premier polypeptide est codé par une molécule d'acide nucléique qui présente la séquence de nucléotides SEQ ID N° : 2 ou 3 du protocole de séquence joint, ou des variantes de celle-ci qui codent pour le même polypeptide en raison de la dégénérescence du code génétique.

8. Protéine de fusion selon l'une des revendications 1 à 7, **caractérisée en ce que** le deuxième polypeptide présente la séquence d'acides aminés SEQ ID N° : 4 du protocole de séquence joint.

9. Protéine de fusion selon l'une des revendications 1 à 8, **caractérisée en ce que** le deuxième polypeptide est codé par une molécule d'acide nucléique qui présente la séquence de nucléotides SEQ ID N° : 5 du protocole de séquence joint ou une variante de celle-ci qui code pour le même polypeptide en raison de la dégénérescence du code génétique.

10. Protéine de fusion selon l'une des revendications 1 à 9, **caractérisée en ce qu'**elle présente la séquence d'acides aminés SEQ ID N° : 6 du protocole de séquence joint.

11. Protéine de fusion selon l'une des revendications 1 à 10, **caractérisée en ce qu'**elle code pour une molécule d'acide nucléique qui présente la séquence de nucléotides SEQ ID N° 7 ou 8 du protocole de séquence joint ou une variante de celle-ci, qui code pour la même protéine de fusion en raison de la dégénérescence du code génétique.

12. Protéine de fusion selon l'une des revendications 1 à 11, **caractérisée en ce que** la protéine de fusion présente en outre un marqueur détectable.

13. Homodimère qui présente la protéine de fusion selon l'une des revendications 1 à 12.

14. Molécule d'acide nucléique qui code pour la protéine de fusion selon l'une des revendications 1 à 12.

15. Molécule d'acide nucléique selon la revendication 14, présentant :
(a) une première séquence de nucléotides qui code pour un polypeptide se liant spécifiquement à oxLDL, et
(b) une deuxième séquence de nucléotides qui code pour un polypeptide entraînant une dimérisation,
**caractérisée en ce que** le premier polypeptide présente la fonction de liaison du LDL de CD68.

16. Molécule d'acide nucléique selon la revendication 14 ou 15, **caractérisée en ce qu'**elle présente la séquence de nucléotides SEQ ID N° : 2 ou 3, du protocole de séquences joint ou des variantes de celle-ci, qui codent pour le même polypeptide en raison de la dégénérescence du code génétique.

17. Molécule d'acide nucléique selon l'une des revendications 14 à 16, **caractérisée en ce qu'**elle présente la séquence de nucléotides SEQ ID N° : 5 du protocole de séquence joint ou une variante de celle-ci qui code pour le même polypeptide en raison de la dégénérescence du code génétique.

18. Molécule d'acide nucléique selon l'une des revendications 14 à 17, **caractérisée en ce que** la séquence de nucléotides SEQ ID N° : 7 ou 8 du protocole de séquence joint ou une variante de celle-ci codent pour le même polypeptide en raison de la dégénérescence du code génétique.

19. Composition pharmaceutique et/ou diagnostique qui présente la protéine de fusion selon l'une des revendications 1 à 12 et/ou l'homodimère selon la revendication 13 et/ou la molécule d'acide nucléique selon l'une des revendications 14 à 18 et éventuellement un support acceptable d'un point de vue pharmaceutique et/ou diagnostic et éventuellement d'autres additifs efficaces d'un point de vue pharmaceutique et/ou diagnostic.

20. Utilisation de la protéine de fusion selon l'une des revendications 1 à 12 et/ou de l'homodimère selon la revendication 13 et/ou de la molécule d'acide nucléique selon l'une des revendications 14 à 18 pour la production d'une composition pharmaceutique et/ou diagnostique pour le traitement et/ou le diagnostic de maladies vasculaires aiguës ou chroniques comprenant l'athérosclérose et les plaques artérioscléreuses.

21. Procédé de production de la protéine de fusion selon l'une des revendications 1 à 12, qui présente les étapes suivantes :
(1) mise à disposition d'une première séquence de nucléotides qui code pour un polypeptide se liant spécifiquement à oxLDL qui présente la fonction de liaison du LDL de CD68,
(2) mise à disposition d'une deuxième séquence de nucléotide qui code pour un polypeptide entraînant une dimérisation,
(3) liaison de la première et de la deuxième séquence de nucléotides pour l'obtention d'une séquence de fusion codant pour la protéine de fusion,
(4) clonage de la séquence de fusion dans un vecteur d'expression,
(5) insertion du vecteur d'expression dans une cellule appropriée pour l'expression,
(6) expression de la protéine de fusion dans la cellule et
(7) isolement de la protéine de fusion de la cellule.


### SEQUENZPROTOKOLL

<110> Eberhard-Karls-Universität, Universitätsklinikum Tübingen
<120> Antientzündliches Fusionsprotein
<130> 5402P362
<160> 8
<170> PatentIn Version 3.3
<210> 1
   <211> 298
   <212> PRT
   <213> Homo sapiens
<400> 1
   Asn Asp Cys Pro His Lys Lys Ser Ala Thr Leu Leu Pro Ser Phe Thr
   1               5                   10                  15
   Val Thr Pro Thr Val Thr Glu Ser Thr Gly Thr Thr Ser His Arg Thr
               20                  25                  30
   Thr Lys Ser His Lys Thr Thr Thr His Arg Thr Thr Thr Thr Gly Thr
           35                  40                  45
   Thr Ser His Gly Pro Thr Thr Ala Thr His Asn Pro Thr Thr Thr Ser
       50                  55                  60
   His Gly Asn Val Thr Val His Pro Thr Ser Asn Ser Thr Ala Thr Ser
   65                  70                  75                  80
   Gln Gly Pro Ser Thr Ala Thr His Ser Pro Ala Thr Thr Ser His Gly
                   85                  90                  95
   Asn Ala Thr Val His Pro Thr Ser Asn Ser Thr Ala Thr Ser Pro Gly
               100                 105                 110
   Phe Thr Ser Ser Ala His Pro Glu Pro Pro Pro Pro Ser Pro Ser Pro
           115                 120                 125
   Ser Pro Thr Ser Lys Glu Thr Ile Gly Asp Tyr Thr Trp Thr Asn Gly
   130 135 140
   Ser Gln Pro Cys Val His Leu Gln Ala Gln Ile Gln Ile Arg Val Met
   145 150 155 160
   Tyr Thr Thr Gln Gly Gly Gly Glu Ala Trp Gly Ile Ser Val Leu Asn
   165 170 175
   Pro Asn Lys Thr Lys Val Gln Gly Ser Cys Glu Gly Ala His Pro His
   180 185 190
   Leu Leu Leu Ser Phe Pro Tyr Gly His Leu Ser Phe Gly Phe Met Gln
   195 200 205
   Asp Leu Gln Gln Lys Val Val Tyr Leu Ser Tyr Met Ala Val Glu Tyr
   210 215 220
   Asn Val Ser Phe Pro His Ala Ala Gln Trp Thr Phe Ser Ala Gln Asn
   225 230 235 240
   Ala Ser Leu Arg Asp Leu Gln Ala Pro Leu Gly Gln Ser Phe Ser Cys
   245 250 255
   Ser Asn Ser Ser Ile Ile Leu Ser Pro Ala Val His Leu Asp Leu Leu
   260 265 270
   Ser Leu Arg Leu Gln Ala Ala Gln Leu Pro His Thr Gly Val Phe Gly
   275 280 285
   Gln Ser Phe Ser Cys Pro Ser Asp Arg Ser
   290 295
<210> 2
   <211> 894
   <212> DNA
   <213> Homo sapiens
<400> 2
   aatgactgtc ctcacaaaaa atcagctact ttgctgccat ccttcacggt gacacccacg 60
   gttacagaga gcactggaac aaccagccac aggactacca agagccacaa aaccaccact 120
   cacaggacaa ccaccacagg caccaccagc cacggaccca cgactgccac tcacaacccc 180
   accaccacca gccatggaaa cgtcacagtt catccaacaa gcaatagcac tgccaccagc 240
   cagggaccct caactgccac tcacagtcct gccaccacta gtcatggaaa tgccacggtt 300
   catccaacaa gcaacagcac tgccaccagc ccaggattca ccagttctgc ccacccagaa 360
   ccacctccac cctctccgag tcctagccca acctccaagg agaccattgg agactacacg 420
   tggaccaatg gttcccagcc ctgtgtccac ctccaagccc agattcagat tcgagtcatg 480
   tacacaaccc agggtggagg agaggcctgg ggcatctctg tactgaaccc caacaaaacc 540
   aaggtccagg gaagctgtga gggtgcccat ccccacctgc ttctctcatt cccctatgga 600
   cacctcagct ttggattcat gcaggacctc cagcagaagg ttgtctacct gagctacatg 660
   gcggtggagt acaatgtgtc cttcccccac gcagcacagt ggacattctc ggctcagaat 720
   gcatcccttc gagatctcca agcacccctg gggcagagct tcagttgcag caactcgagc 780
   atcattcttt caccagctgt ccacctcgac ctgctctccc tgaggctcca ggctgctcag 840
   ctgccccaca caggggtctt tgggcaaagt ttctcctgcc ccagtgaccg gtcc 894
<210> 3
   <211> 894
   <212> DNA
   <213> Homo sapiens
<400> 3
   aatgactgtc ctcacaaaaa atcagctact ttgctgccat ccttcacggt gacacccacg 60
   gttacagaga gcactggaac aaccagccac aggactacca agagccacaa aaccaccact 120
   cacaggacaa ccaccacagg caccaccagc cacggaccca cgactgccac tcacaacccc 180
   accaccacca gccatggaaa cgtcacagtt catccaacaa gcaatagcac tgccaccagc 240
   cagggaccct caactgccac tcacagtcct gccaccacta gtcatggaaa tgccacggtt 300
   catccaacaa gcaacagcac tgccaccagc ccaggattca ccagttctgc ccacccagaa 360
   ccacctccac cctctccgag tcctagccca acctccaagg agaccattgg agactacacg 420
   tggaccaatg gttcccagcc ctgtgtccac ctccaagccc agattcagat tcgagtcatg 480
   tacacaaccc agggtggagg agaggcctgg ggcatctctg tactgaaccc caacaaaacc 540
   aaggtccagg gaagctgtga gggtgcccat ccccacctgc ttctctcatt cccctatgga 600
   cacctcagct ttggattcat gcaggacctc cagcagaagg ttgtctacct gagctacatg 660
   gcggtggagt acaatgtgtc cttcccccac gcagcaaagt ggacattctc ggctcagaat 720
   gcatcccttc gagatctcca agcacccctg gggcagagct tcagttgcag caactcgagc 780
   atcattcttt caccagctgt ccacctcgac ctgctctccc tgaggctcca ggctgctcag 840
   ctgccccaca caggggtctt tgggcaaagt ttctcctgcc ccagtgaccg gtcc 894
<210> 4
   <211> 232
   <212> PRT
   <213> Künstliche Sequenz
<400> 4
   Glu Ser Lys Ser Cys Asp Lys Thr His Thr Cys Pro Pro Cys Pro Ala
   1               5                   10                  15
   Pro Glu Ala Ala Ala Ala Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Ser Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 5
   <211> 699
   <212> DNA
   <213> Künstliche Sequenz
<400> 5
   gagtctaagt cctgtgataa gactcatacc tgccccccct gcccagcacc cgaggcagca      60
   gccgccccct cagtgtttct cttccctcca aaacccaagg ataccctgat gatcagccgt     120
   acacctgagg tcacctgcgt agtcgtcgat gtgtctcacg aggacccgga ggtgaagttt    180
   aattggtatg tggacggggt agaagtgcat aatgccaaga ctaaacctcg agaggaacaa    240
   tataactcca cctatagggt ggtcagcgtt ctcacggtcc ttcaccagga ctggttgaat    300
   ggaaaggaat acaagtgtaa ggtgagcaac aaagccctgc ccgcttccat agaaaagaca    360
   atctccaaag ctaaagggca gccacgggaa cctcaggtgt acaccctgcc gcctagcaga    420
   gatgagctca caaagaacca ggtgtctctg acatgcttgg tgaagggttt ctatccttcg    480
   gacattgccg ttgagtggga aagtaacggc cagcctgaga ataactacaa gaccacacca    540
   cccgttcttg actctgatgg gagtttcttt ttgtacagta agttaactgt cgacaaatca    600
   cgctggcagc aaggaaatgt tttctcttgt tccgtgatgc acgaggcact gcacaaccat    660
   tacactcaga aatccctgag cctatcacca ggcaaataa                          699
<210> 6
   <211> 533
   <212> PRT
   <213> Künstliche Sequenz
<220>
   <223> Fusionsprotein
<400> 6
   Asn Asp Cys Pro His Lys Lys Ser Ala Thr Leu Leu Pro Ser Phe Thr
   1               5                   10                  15
   Val Thr Pro Thr Val Thr Glu Ser Thr Gly Thr Thr Ser His Arg Thr
               20                  25                  30
   Thr Lys Ser His Lys Thr Thr Thr His Arg Thr Thr Thr Thr Gly Thr
           35                  40                  45
   Thr Ser His Gly Pro Thr Thr Ala Thr His Asn Pro Thr Thr Thr Ser
       50                  55                  60
   His Gly Asn Val Thr Val His Pro Thr Ser Asn Ser Thr Ala Thr Ser
   65                  70                  75                  80
   Gln Gly Pro Ser Thr Ala Thr His Ser Pro Ala Thr Thr Ser His Gly
                   85                  90                  95
   Asn Ala Thr Val His Pro Thr Ser Asn Ser Thr Ala Thr Ser Pro Gly
               100                 105                 110
   Phe Thr Ser Ser Ala His Pro Glu Pro Pro Pro Pro Ser Pro Ser Pro
           115                 120                 125
   Ser Pro Thr Ser Lys Glu Thr Ile Gly Asp Tyr Thr Trp Thr Asn Gly
       130                 135                 140
   Ser Gln Pro Cys Val His Leu Gln Ala Gln Ile Gln Ile Arg Val Met
   145                 150                 155                 160
   Tyr Thr Thr Gln Gly Gly Gly Glu Ala Trp Gly Ile Ser Val Leu Asn
                   165                 170                 175
   Pro Asn Lys Thr Lys Val Gln Gly Ser Cys Glu Gly Ala His Pro His
               180                 185                 190
   Leu Leu Leu Ser Phe Pro Tyr Gly His Leu Ser Phe Gly Phe Met Gln
           195                 200                 205
   Asp Leu Gln Gln Lys Val Val Tyr Leu Ser Tyr Met Ala Val Glu Tyr
       210                 215                 220
   Asn Val Ser Phe Pro His Ala Ala Gln Trp Thr Phe Ser Ala Gln Asn
   225 230 235 240
   Ala Ser Leu Arg Asp Leu Gln Ala Pro Leu Gly Gln Ser Phe Ser Cys
   245 250 255
   Ser Asn Ser Ser Ile Ile Leu Ser Pro Ala Val His Leu Asp Leu Leu
   260 265 270
   Ser Leu Arg Leu Gln Ala Ala Gln Leu Pro His Thr Gly Val Phe Gly
   275 280 285
   Gln Ser Phe Ser Cys Pro Ser Asp Arg Ser Gly Gly Arg Glu Ser Lys
   290 295 300
   Ser Cys Asp Lys Thr His Thr Cys Pro Pro Cys Pro Ala Pro Glu Ala
   305 310 315 320
   Ala Ala Ala Pro Ser Val Phe Leu Phe Pro Pro Lys Pro Lys Asp Thr
   325 330 335
   Leu Met Ile Ser Arg Thr Pro Glu Val Thr Cys Val Val Val Asp Val
   340 345 350
   Ser His Glu Asp Pro Glu Val Lys Phe Asn Trp Tyr Val Asp Gly Val
   355 360 365
   Glu Val His Asn Ala Lys Thr Lys Pro Arg Glu Glu Gln Tyr Asn Ser
   370 375 380
   Thr Tyr Arg Val Val Ser Val Leu Thr Val Leu His Gln Asp Trp Leu
   385 390 395 400
   Asn Gly Lys Glu Tyr Lys Cys Lys Val Ser Asn Lys Ala Leu Pro Ala
   405 410 415
   Ser Ile Glu Lys Thr Ile Ser Lys Ala Lys Gly Gln Pro Arg Glu Pro
   420 425 430
   Gln Val Tyr Thr Leu Pro Pro Ser Arg Asp Glu Leu Thr Lys Asn Gln
   435 440 445
   Val Ser Leu Thr Cys Leu Val Lys Gly Phe Tyr Pro Ser Asp Ile Ala
   450 455 460
   Val Glu Trp Glu Ser Asn Gly Gln Pro Glu Asn Asn Tyr Lys Thr Thr
   465                 470                 475                 480
   Pro Pro Val Leu Asp Ser Asp Gly Ser Phe Phe Leu Tyr Ser Lys Leu
                   485                 490                 495
   Thr Val Asp Lys Ser Arg Trp Gln Gln Gly Asn Val Phe Ser Cys Ser
               500                 505                 510
   Val Met His Glu Ala Leu His Asn His Tyr Thr Gln Lys Ser Leu Ser
           515                 520                 525
   Leu Ser Pro Gly Lys
       530
<210> 7
   <211> 1602
   <212> DNA
   <213> Künstliche Sequenz
<220>
   <223> Fusionsprotein
<400> 7
   aatgactgtc ctcacaaaaa atcagctact ttgctgccat ccttcacggt gacacccacg      60
   gttacagaga gcactggaac aaccagccac aggactacca agagccacaa aaccaccact     120
   cacaggacaa ccaccacagg caccaccagc cacggaccca cgactgccac tcacaacccc     180
   accaccacca gccatggaaa cgtcacagtt catccaacaa gcaatagcac tgccaccagc     240
   cagggaccct caactgccac tcacagtcct gccaccacta gtcatggaaa tgccacggtt     300
   catccaacaa gcaacagcac tgccaccagc ccaggattca ccagttctgc ccacccagaa     360
   ccacctccac cctctccgag tcctagccca acctccaagg agaccattgg agactacacg     420
   tggaccaatg gttcccagcc ctgtgtccac ctccaagccc agattcagat tcgagtcatg     480
   tacacaaccc agggtggagg agaggcctgg ggcatctctg tactgaaccc caacaaaacc     540
   aaggtccagg gaagctgtga gggtgcccat ccccacctgc ttctctcatt cccctatgga     600
   cacctcagct ttggattcat gcaggacctc cagcagaagg ttgtctacct gagctacatg     660
   gcggtggagt acaatgtgtc cttcccccac gcagcacagt ggacattctc ggctcagaat     720
   gcatcccttc gagatctcca agcacccctg gggcagagct tcagttgcag caactcgagc     780
   atcattcttt caccagctgt ccacctcgac ctgctctccc tgaggctcca ggctgctcag     840
   ctgccccaca caggggtctt tgggcaaagt ttctcctgcc ccagtgaccg gtccggcggc     900
   cgcgagtcta agtcctgtga taagactcat acctgccccc cctgcccagc acccgaggca     960
   gcagccgccc cctcagtgtt tctcttccct ccaaaaccca aggataccct gatgatcagc    1020
   cgtacacctg aggtcacctg cgtagtcgtc gatgtgtctc acgaggaccc ggaggtgaag    1080
   tttaattggt atgtggacgg ggtagaagtg cataatgcca agactaaacc tcgagaggaa    1140
   caatataact ccacctatag ggtggtcagc gttctcacgg tccttcacca ggactggttg    1200
   aatggaaagg aatacaagtg taaggtgagc aacaaagccc tgcccgcttc catagaaaag    1260
   acaatctcca aagctaaagg gcagccacgg gaacctcagg tgtacaccct gccgcctagc    1320
   agagatgagc tcacaaagaa ccaggtgtct ctgacatgct tggtgaaggg tttctatcct    1380
   tcggacattg ccgttgagtg ggaaagtaac ggccagcctg agaataacta caagaccaca    1440
   ccacccgttc ttgactctga tgggagtttc tttttgtaca gtaagttaac tgtcgacaaa    1500
   tcacgctggc agcaaggaaa tgttttctct tgttccgtga tgcacgaggc actgcacaac    1560
   cattacactc agaaatccct gagcctatca ccaggcaaat aa                       1602
<210> 8
   <211> 1602
   <212> DNA
   <213> Künstliche Sequenz
<220>
   <223> Fusionsprotein
<400> 8
   aatgactgtc ctcacaaaaa atcagctact ttgctgccat ccttcacggt gacacccacg      60
   gttacagaga gcactggaac aaccagccac aggactacca agagccacaa aaccaccact     120
   cacaggacaa ccaccacagg caccaccagc cacggaccca cgactgccac tcacaacccc     180
   accaccacca gccatggaaa cgtcacagtt catccaacaa gcaatagcac tgccaccagc     240
   cagggaccct caactgccac tcacagtcct gccaccacta gtcatggaaa tgccacggtt     300
   catccaacaa gcaacagcac tgccaccagc ccaggattca ccagttctgc ccacccagaa     360
   ccacctccac cctctccgag tcctagccca acctccaagg agaccattgg agactacacg    420
   tggaccaatg gttcccagcc ctgtgtccac ctccaagccc agattcagat tcgagtcatg    480
   tacacaaccc agggtggagg agaggcctgg ggcatctctg tactgaaccc caacaaaacc    540
   aaggtccagg gaagctgtga gggtgcccat ccccacctgc ttctctcatt cccctatgga    600
   cacctcagct ttggattcat gcaggacctc cagcagaagg ttgtctacct gagctacatg    660
   gcggtggagt acaatgtgtc cttcccccac gcagcaaagt ggacattctc ggctcagaat    720
   gcatcccttc gagatctcca agcacccctg gggcagagct tcagttgcag caactcgagc    780
   atcattcttt caccagctgt ccacctcgac ctgctctccc tgaggctcca ggctgctcag    840
   ctgccccaca caggggtctt tgggcaaagt ttctcctgcc ccagtgaccg gtccggcggc    900
   cgcgagtcta agtcctgtga taagactcat acctgccccc cctgcccagc acccgaggca    960
   gcagccgccc cctcagtgtt tctcttccct ccaaaaccca aggataccct gatgatcagc   1020
   cgtacacctg aggtcacctg cgtagtcgtc gatgtgtctc acgaggaccc ggaggtgaag   1080
   tttaattggt atgtggacgg ggtagaagtg cataatgcca agactaaacc tcgagaggaa   1140
   caatataact ccacctatag ggtggtcagc gttctcacgg tccttcacca ggactggttg   1200
   aatggaaagg aatacaagtg taaggtgagc aacaaagccc tgcccgcttc catagaaaag   1260
   acaatctcca aagctaaagg gcagccacgg gaacctcagg tgtacaccct gccgcctagc   1320
   agagatgagc tcacaaagaa ccaggtgtct ctgacatgct tggtgaaggg tttctatcct   1380
   tcggacattg ccgttgagtg ggaaagtaac ggccagcctg agaataacta caagaccaca   1440
   ccacccgttc ttgactctga tgggagtttc tttttgtaca gtaagttaac tgtcgacaaa   1500
   tcacgctggc agcaaggaaa tgttttctct tgttccgtga tgcacgaggc actgcacaac   1560
   cattacactc agaaatccct gagcctatca ccaggcaaat aa                      1602